(19) **Europäisches Patentamt / European Patent Office / Office européen des brevets**

(11) **EP 3 845 887 B1**

(12) **EUROPEAN PATENT SPECIFICATION**

(45) Date of publication and mention
of the grant of the patent:
**01.05.2024 Bulletin 2024/18**

(21) Application number: **19856288.6**

(22) Date of filing: **30.08.2019**

(51) International Patent Classification (IPC):
**G01N 21/78** (2006.01)   **G01N 21/64** (2006.01)
**G01N 30/88** (2006.01)   **G01N 33/50** (2006.01)

(52) Cooperative Patent Classification (CPC):
**G01N 21/6428**

(86) International application number:
**PCT/JP2019/034101**

(87) International publication number:
**WO 2020/045621 (05.03.2020 Gazette 2020/10)**

(54) **SKIN SENSITIZATION MEASURING METHOD**

VERFAHREN ZUR MESSUNG DER HAUTSENSIBILISIERUNG

PROCÉDÉ DE MESURE DE SENSIBILISATION DE LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.08.2018   JP 2018163423**
**25.03.2019   JP 2019056675**

(43) Date of publication of application:
**07.07.2021 Bulletin 2021/27**

(73) Proprietor: **FUJIFILM Corporation
Tokyo 106-8620 (JP)**

(72) Inventors:
 • **FUJITA Masaharu**
  **Ashigarakami-gun, Kanagawa 258-8577 (JP)**
 • **KASAHARA Toshihiko**
  **Ashigarakami-gun, Kanagawa 258-8577 (JP)**
 • **YAMAMOTO Yusuke**
  **Ashigarakami-gun, Kanagawa 258-8577 (JP)**

(74) Representative: **Hoffmann Eitle
Patent- und Rechtsanwälte PartmbB
Arabellastraße 30
81925 München (DE)**

(56) References cited:
 **WO-A2-2015/200870    JP-A- 2009 222 466
 JP-A- 2011 059 102    JP-A- 2014 037 995**

 • **FUJITA MASAHARU ET AL: "Development of a prediction method for skin sensitization using novel cysteine and lysine derivatives", JOURNAL OF PHARMACOLOGICAL AND TOXICOLOGICAL METHODS, ELSEVIER, NEW YORK, NY, US, vol. 70, no. 1, 10 June 2014 (2014-06-10), pages 94-105, XP029036887, ISSN: 1056-8719, DOI: 10.1016/J.VASCN.2014.06.001**
 • **Fujita Masaharu: "In chemico alternative method DPRA and ADRA for skin sensitization -principle and issues", Journal of Toxicoloical Sciences, vol. 43, June 2018 (2018-06), page S167, XP009526191, DOI: 10.14869/toxpt.45.1.0_W7-2**
 • **Fujita Masaharu; Yamamoto Yusuke; Tahara Haruna; Kasahara Toshihiko; Jimbo Yoshihiro; Hioki Takanori: "Development of a prediction method for skin sensitization using novel cysteine and lysine derivatives", Journal of Pharmacological and Toxicological Methods, vol. 70, no. 1, 10 June 2014 (2014-06-10), pages 94-105, XP029036887, ISSN: 1056-8719, DOI: 10.1016/j.vascn.2014.06.001**

**Description**

Field of the Invention

The present invention relates to a method for determining, with a fluorescence detector, skin allergenicity of a mixture containing two or more test substances.

Description of the Related Art

Skin allergenicity (allergenicity) involves, in some cases, not only symptoms such as blisters or erythema locally occurring at spots exposed to a substance, but also anaphylaxis, which is a systemic allergic reaction that is severe and threatens the life. In addition, skin allergenicity, which, after the onset, causes, for example, requirement of care for avoiding the exposure for a long term, is considered as one of serious toxic properties. It is important that chemical substances included in products such as pharmaceuticals, agricultural chemicals, and cosmetics are substances that do not cause allergic reactions; in the development of products, chemical substances employed need to be checked for skin allergenicity.

As methods for evaluating skin allergenicity of chemical substances, there are well-known test methods using guinea pigs. Test methods such as GPMT (Guinea Pig Maximisation Test) using adjuvant and Buehler Test, which is a non-adjuvant test, have been widely used for many years.

Animal experiments involve many problems such as cumbersome operations, tests for long terms, and huge test expenses, and there are also ethical and social needs such as animal welfare, so that alternative methods not using animals are being developed. In particular, development of methods for testing for skin allergenicity that causes severe symptoms is a matter of urgent necessity, and in vitro tests using cultured cells and in chemico tests using chemical reactions have been developed.

Such in vitro tests that are known are ARE-Nrf2 luciferase KeratinoSens™ test method (KeratinoSens is a registered trademark), LuSens (ARE-NrF2 lusiferase LuSens test method), h-CLAT (human Cell Line Activation Test), U-SENS (Myeloid U937 Skin Sensitization Test), and IL-8 Luc assay.

The in chemico tests using chemical reactions, which do not use cultured cells, provides many advantages such as elimination of the necessity of special techniques, knowledge, and equipment. For example, Non-Patent Documents 1 and 2 describe a method of using two peptide species (cysteine peptide and lysine peptide) as nucleophilic reagents. In addition, Patent Documents 1 and 2 describe reagents for determining skin allergenicity and methods for determining skin allergenicity in which an aryl-ring-introduced cysteine derivative and an aryl-ring-introduced lysine derivative are used as nucleophilic reagents.

Prior Art Documents

Non-Patent Documents

Non-Patent Document 1: Gerberick, G. F. et al. (2004). Development of a peptide reactivity assay for screening contact allergens. Toxicological Sciences, 81(2), 332-43
Non-Patent Document 2: Gerberick, G. F. et al. (2007). Quantification of chemical peptide reactivity for screening contact allergens: a classification tree model approach. Toxicological Sciences, 97(2), 417-27

Patent Documents

Patent Document 1: JP2011-59102A
Patent Document 2: JP2014-37995A

**SUMMARY OF THE INVENTION**

The methods for determining skin allergenicity described in Non-Patent Documents 1 and 2 are good test methods that are simple and have high prediction accuracy; however, the two peptides used have themselves low molar absorption coefficients, and are detectable only at a short wavelength of 220 nm, so that, in quantification of the remaining percent of the peptides by HPLC-UV method (high-performance liquid chromatography-ultraviolet method), the quantification sensitivity is low and phenomena such as coelution of a peptide and a test substance tend to occur, and hence the quantification is often difficult to achieve, which is problematic. The reagents for determining skin allergenicity described in Patent Documents 1 and 2 are good reagents that address such problems.

The methods for determining skin allergenicity described in Non-Patent Documents 1 and 2 are applicable when the chemical structure of the test substance serving as the subject is known; and since the determination methods require preparation of a 100 mmol/L solution of the test substance, the molecular weight of the test substance is necessary. In summary, the methods for determining skin allergenicity described in Non-Patent Documents 1 and 2 cannot be used for evaluating substances (including mixtures) having unknown molecular weights. The methods for determining skin allergenicity described in Patent Documents 1 and 2 are basically based on the same principle as in the methods for determining skin allergenicity described in Non-Patent Documents 1 and 2. Patent Documents 1 and 2 describe determination for a single test substance that has a known chemical structure.

However, the test substance to be subjected to prediction for skin allergenicity is not necessarily a single substance, but is conversely often a multi-component mixtures composed of a plurality of components. In such cases of evaluating the skin allergenicity of a multi-component mixture, the following requirements need to be satisfied: evaluation is achieved even for components having unknown molecular weights, and quantification is achieved in chromatography measurement without occurrence of coelution of a nucleophilic reagent and two or more test substances.

An object of the present invention is to provide a method for determining skin allergenicity of a mixture containing two or more test substances, and a reagent for determining the skin allergenicity.

The inventors of the present invention performed thorough studies on how to achieve the above-described object. As a result, they have found that, by causing a reaction between an organic compound that has at least one thiol group or amino group and emits fluorescence, and a mixture containing two or more test substances, and by determining the amount of the organic compound after the reaction or the amount of the reaction product by an optical measurement using a fluorescence detector, the skin allergenicity of the mixture containing two or more test substances is accurately determined. The present invention has been accomplished on the basis of these findings.

## BRIEF DESCRIPTION OF THE DRAWINGS

Fig. 1 includes HPLC charts by UV detection and fluorescence detection for a green-tea extract (undiluted solution) (not including any nucleophilic reagent);
Fig. 2 includes HPLC charts by UV detection and fluorescence detection for a 1/10 diluted solution of a green-tea extract (not including any nucleophilic reagent);
Fig. 3 includes HPLC charts by UV detection and fluorescence detection for a 1/100 diluted solution of a green-tea extract (not including any nucleophilic reagent);
Fig. 4 includes HPLC charts by UV detection and fluorescence detection for a 1/1000 diluted solution of a green-tea extract (not including any nucleophilic reagent);
Fig. 5 includes HPLC charts by UV detection and fluorescence detection for reaction solutions of a green-tea extract (undiluted solution) and a nucleophilic reagent (NAC or NAL);
Fig. 6 includes HPLC charts by UV detection and fluorescence detection for reaction solutions of a 1/10 diluted solution of a green-tea extract and a nucleophilic reagent (NAC or NAL);
Fig. 7 includes HPLC charts by UV detection and fluorescence detection for reaction solutions of a 1/100 diluted solution of a green-tea extract and a nucleophilic reagent (NAC or NAL);
Fig. 8 includes HPLC charts by UV detection and fluorescence detection for reaction solutions of a 1/1000 diluted solution of a green-tea extract and a nucleophilic reagent (NAC or NAL);
Fig. 9 includes HPLC charts by UV detection and fluorescence detection for an aloe model extract (20-component solution mixture) (undiluted solution) (not including any nucleophilic reagent);
Fig. 10 includes HPLC charts by UV detection and fluorescence detection for a 1/10 diluted solution (not including any nucleophilic reagent) of an aloe model extract (20-component solution mixture);
Fig. 11 includes HPLC charts by UV detection and fluorescence detection for reaction solutions of an aloe model extract (20-component solution mixture) (undiluted solution) and a nucleophilic reagent (NAC or NAL);
Fig. 12 includes HPLC charts by UV detection and fluorescence detection for reaction solutions of a 1/10 diluted solution of an aloe model extract (20-component solution mixture) and a nucleophilic reagent (NAC or NAL);
Fig. 13 includes HPLC charts by UV detection and fluorescence detection for $\beta$-sitosterol (undiluted solution) (not including any nucleophilic reagent);
Fig. 14 includes HPLC charts by UV detection and fluorescence detection for a 1/10 diluted solution of $\beta$-sitosterol (not including any nucleophilic reagent);
Fig. 15 includes HPLC charts by UV detection and fluorescence detection for reaction solutions of $\beta$-sitosterol

(undiluted solution) and a nucleophilic reagent (NAC or NAL);

Fig. 16 includes HPLC charts by UV detection and fluorescence detection for reaction solutions of a 1/10 diluted solution of β-sitosterol and a nucleophilic reagent (NAC or NAL);

Fig. 17 includes HPLC charts by UV detection and fluorescence detection for protocatechuic acid (undiluted solution) (not including any nucleophilic reagent);

Fig. 18 includes HPLC charts by UV detection and fluorescence detection for a 1/10 diluted solution of protocatechuic acid (not including any nucleophilic reagent);

Fig. 19 includes HPLC charts by UV detection and fluorescence detection for reaction solutions of protocatechuic acid (undiluted solution) and a nucleophilic reagent (NAC or NAL);

Fig. 20 includes HPLC charts by UV detection and fluorescence detection for reaction solutions of a 1/10 diluted solution of protocatechuic acid and a nucleophilic reagent (NAC or NAL); and

Fig. 21 includes HPLC charts by UV detection and fluorescence detection for a nucleophilic reagent (NAC or NAL).

## DESCRIPTION OF THE PREFERRED EMBODIMENTS

In this Specification, 'a value "to" a value' is intended to include these values as the lower limit value and the upper limit value.

The present invention relates to a method for determining skin allergenicity, the method including causing a reaction between an organic compound that has at least one thiol group or amino group and emits fluorescence, and a mixture containing two or more test substances, and determining an amount of the organic compound after the reaction or an amount of a product of the reaction by an optical measurement using a fluorescence detector.

The method of the present invention is defined in claim 1.

As described above, in the cases of evaluating the skin allergenicity of a multi-component mixture, the following requirements need to be satisfied: evaluation is achieved even for components having unknown molecular weights, and quantification is achieved in chromatography measurement without occurrence of coelution of a nucleophilic reagent and two or more test substances.

In the present invention, by using, as a nucleophilic reagent, an organic compound that has at least one thiol group or amino group and emits fluorescence, the nucleophilic reagent can be detected at high sensitivity, which is advantageous. According to the present invention, the organic compound used as the nucleophilic reagent is selected from a N-(arylalkylcarbonyl)cysteine or a-N-(arylalkylcarbonyl)lysine. Either of the compounds is detectable for fluorescence and, by using this, it is quantified as being completely distinguished from the test substances. Thus, the present invention enables determination of the skin allergenicity of a mixture including two or more test substances, which cannot be evaluated by the methods for determining skin allergenicity described in Gerberick, G. F. et al. (2004). Development of a peptide reactivity assay for screening contact allergens. Toxicological Sciences, 81(2), 332-43 and Gerberick, G. F. et al. (2007). Quantification of chemical peptide reactivity for screening contact allergens: a classification tree model approach. Toxicological Sciences, 97(2), 417-27.

In the present invention, the meaning of determination of skin allergenicity includes testing for skin allergenicity, and includes judgement of the presence or absence of skin allergenicity based on predetermined criteria and quantitative measurement of skin allergenicity.

Skin allergenicity causes a complicated process composed of many stages, resulting in symptoms. Of these, the first stage is that the test substance permeates the skin and then forms covalent bonds with protein within the skin. Thus, the covalent bondability is evaluated to thereby inferentially predict whether or not the test substance serving as the subject has skin allergenicity. The reaction between the protein within the skin and the test substance is known to be composed of about five organic chemical reactions. Regarding amino acids involving these five reactions, the SH group of cysteine and an $NH_2$ group of lysine are known. Thus, in Examples of the present invention, compounds provided by introducing, to the N terminus of cysteine or lysine, an aryl ring having high molar absorption coefficient in the UV region were used as nucleophilic reagents, and these two nucleophilic reagents are caused to react with test substances, and the unreacted nucleophilic reagents were quantified. As described above, the method for determining skin allergenicity according to the present invention is a test method of calculating the reactivity of a nucleophilic reagent and test substances, to predict skin allergenicity.

In the present invention, the mixture containing two or more test substances means a mixture that includes, as components corresponding to main components, the two or more test substances, and does not mean a mixture that includes a single test substance and various impurities.

Non-limiting specific examples of the mixture in the present invention include at least one selected from the group consisting of perfume, essential oil, polymers, pharmaceuticals, agricultural chemicals, foods, chemical products, and plant extracts composed of natural-product-derived components.

In the present invention, an organic compound selected from a N-(arylalkylcarbonyl)cysteine or a-N-(arylalkylcarbonyl)lysine (also referred to as a nucleophilic reagent) is used.

The organic compound that has at least one thiol group or amino group and emits fluorescence is preferably an organic compound that emits fluorescence in 200 to 400 nm, and has a molar absorption coefficient at a maximal absorption wavelength of 10 L/mol·cm or more and 500000 L/mol·cm or less.

The organic compound that has at least one thiol group and emits fluorescence is preferably a compound that, itself or in the form of solution, exhibits absorption in a wavelength range of 190 to 2500 nm, more preferably exhibits absorption in a wavelength range of 200 to 700 nm, more preferably a compound that has the maximal absorption in such a wavelength range. The organic compound that has at least one thiol group and emits fluorescence is preferably a compound that has absorption having a molar absorption coefficient (L/mol·cm) of 10 or more at the maximal absorption, more preferably a compound that has absorption having a molar absorption coefficient of 100 or more, particularly preferably a compound that has absorption having the maximal absorption in a wavelength range of 200 to 700 nm and having, at the maximal absorption, a molar absorption coefficient of 100 or more.

Specific examples of the organic compound used in the method of the present invention include N-(2-phenylacetyl)cysteine and N-[2-(naphthalen-1-yl)acetyl]cysteine.

The organic compound that has at least one thiol group and emits fluorescence can be produced by a publicly known method. For example, N-(2-phenylacetyl)cysteine can be synthesized by a method described in Paragraphs 0015 to 0017 of JP2011-59102A.

N-[2-(naphthalen-1-yl)acetyl]cysteine can be synthesized by the following method.

1-Naphthylacetic acid (50 g) is dissolved in 270 mL of toluene; while N,N-dimethylformamide (DMF) is added dropwise, 95.8 g of thionyl chloride is added dropwise at 20°C. A reaction was caused at 60°C for 2 hours, and subsequently cooling is performed; 200 mL of toluene is added, and then drying under a reduced pressure is performed to obtain 1-naphthylacetyl chloride (56 g).

To an aqueous solution of 14 g of sodium hydroxide and 350 mL of water, 20 g of L-cystine is added and dissolved. The solution is cooled with an ice water bath, and 8.76 g of 1-naphthylacetyl chloride is added dropwise. The reaction mixture is stirred at 20°C for 2 hours, and cooled; subsequently, 16.7 mL of concentrated hydrochloric acid is added. About 700 mL of ethyl acetate is added; crystals are collected by filtration, and dried under a reduced pressure to obtain N,N'-bis(1-naphthylacetyl)cystine (39.2 g).

To a mixture of 20 g of N,N'-bis(1-naphthylacetyl)cysteine, 20 g of zinc powder, and 500 mL of methanol under purging with nitrogen, 120 mL of trifluoroacetic acid is added dropwise over 2 hours. The reaction mixture is stirred for 2 hours, and subsequently extracted with 500 mL of ethyl acetate; the organic layer is dried with magnesium sulfate, subsequently filtered, and concentrated. The resultant residue is recrystallized from ethyl acetate, and dried to obtain N-[2-(naphthalen-1-yl)acetyl]cysteine (5.2 g).

N-[2-(naphthalen-1-yl)acetyl]cysteine emits fluorescence in 200 to 400 nm, exhibits maximal absorption at 281 nm, has a molar absorption coefficient of about 7000 (L/mol·cm), and has a maximum fluorescence wavelength of 335 nm.

The organic compound that has at least one amino group and emits fluorescence, which has an amino group, has reactivity even to skin allergenic substances that have low reactivity to cysteine residues, can be measured using a general-purpose simple analysis device, and has sufficient solubility and stability even in a reaction solution used for dissolving hydrophobic chemical substances and having a high organic solvent ratio.

The organic compound that has at least one amino group and emits fluorescence is preferably a compound that, itself or in the form of solution, exhibits absorption in the wavelength range of 190 to 2500 nm, more preferably exhibits absorption in the wavelength range of 200 to 700 nm, more preferably a compound that has the maximal absorption in such a wavelength range. The organic compound that has at least one amino group and emits fluorescence is preferably a compound that has a molar absorption coefficient (L/mol·cm) at the maximal absorption wavelength of 10 or more and 500000 or less, more preferably a compound that has a molar absorption coefficient (L/mol·cm) at the maximal absorption wavelength of 10 to 2000, still more preferably a compound that has a molar absorption coefficient at the maximal absorption wavelength of 100 to 2000, particularly preferably a compound that has the maximal absorption in a wavelength range of 200 to 700 nm, and has a molar absorption coefficient at the maximal absorption wavelength of 100 to 2000.

Incidentally, the molar absorption coefficient ($\varepsilon$) is given by the following formula:

$$\varepsilon = D/(c \cdot d)$$

where D represents the absorbance of the solution, c represents the molarity (mol/L) of the solute, and d represents the thickness of the solution layer (optical path length) (cm). The molar absorption coefficient can be determined by using a commercially available spectrophotometer to measure the absorption spectrum or absorbance.

The organic compound that has at least one amino group and emits fluorescence, from the viewpoint of reactivity to the test substances, preferably has a primary amino group, more preferably is a lysine derivative having an $\varepsilon$-amino group. The lysine derivative is, for example, a lysine derivative in which the carboxyl group and/or $\alpha$-amino group of

lysine is modified with at least one substituent.

The organic compound that has at least one amino group and emits fluorescence preferably has an aromatic group. Examples of the aromatic group include hydrocarbon aromatic groups such as a phenyl group and a naphthyl group, and aromatic groups having a hetero element such as a pyridyl group, a furyl group, and a thiophenyl group. The aromatic group is preferably a phenyl group or a naphthyl group.

Examples of the organic compound that has at least one amino group and emits fluorescence include lysine derivatives having an $\varepsilon$-amino group and an aromatic group.

More specifically, examples of the organic compound that has at least one amino group and emits fluorescence include compounds in which, to the $\alpha$-amino group or carboxyl group of lysine, an aryl group such as a benzene ring or a naphthalene ring is bonded via, for example, an amide bond. Of these, $\alpha$-N-(arylalkylcarbonyl)lysine is particularly preferred. In N-(arylalkylcarbonyl)lysine, the aryl group may have 6 to about 16 carbon atoms. Examples of the aryl group include a benzene ring and a naphthalene ring. The alkylcarbonyl group may have 2 to about 11 carbon atoms. The alkyl group bonded to the carbonyl group may be linear, branched, or cyclic, and examples include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, a 2-ethylhexyl group, and a cyclopropyl group. Specific examples of the organic compound included in a reagent for determining skin allergenicity according to the present invention include $\alpha$-N-(2-phenylacetyl)lysine (hereafter, also referred to as PAL) and $\alpha$-N-[2-(naphthalen-1-yl)acetyl]lysine (hereafter, also referred to as NAL).

The organic compound that has at least one amino group and emits fluorescence can be produced by a publicly known method. For example, PAL and NAL can be synthesized by a method described in Paragraphs 0025 to 0031 of JP2014-37995A.

$\alpha$-N-(2-phenylacetyl)lysine emits fluorescence in 200 to 400 nm, and has a molar absorption coefficient at the maximal absorption wavelength (about 255 nm) of about 200 L/mol·cm.

$\alpha$-N-[2-(naphthalen-1-yl)acetyl]lysine emits fluorescence in 200 to 400 nm, has a molar absorption coefficient at the maximal absorption wavelength (about 280 nm) of about 400 L/mol·cm, and has a maximum fluorescence wavelength of 332 nm.

A reagent for determining skin allergenicity as used the present invention may be composed of the above-described organic compound alone, or may include, in addition to the above-described organic compound serving as a measurement basis, one or two or more additives. Examples of the additives include pH adjusting agents, stabilizing agents, and chelating agents. A reagent for determining skin allergenicity may be a reagent prepared by dissolving the above-described measurement basis and, as needed, the above-described additives in, for example, water, an aqueous buffer solution, an organic solvent, or a solvent mixture of one of the foregoing. A reagent for determining skin allergenicity may be provided in any of forms of solution, liquid, or solid (for example, powder, granules, freeze-dried products, or tablets).

A reagent for determining skin allergenicity may be used, for example, in the form of being dissolved in an aqueous buffer solution including an organic acid salt such as ammonium acetate or an inorganic salt such as phosphate, or water, or a solvent mixture of the foregoing and an organic solvent, such that the concentration of the organic compound is, for example, about 0.01 μmol/L to about 1 mol/L, ordinarily about 0.1 mmol/L to about 500 mmol/L.

In a case where the molarity of the test substance can be adjusted, it may be dissolved in, for example, water, an organic solvent such as methanol, ethanol, acetonitrile, or acetone, or a solvent mixture of the foregoing (a solvent mixture of water and an organic solvent, or a solvent mixture of two or more organic solvents) such that the concentration becomes, for example, about 0.01 μmol/L to about 1 mol/L, ordinarily about 0.1 mmol/L to about 500 mmol/L, or about 1 mmol/L to about 500 mmol/L. Subsequently, the above-described organic compound serving as a measurement basis of a reagent for determining skin allergenicity may be mixed with the test substance solution such that the molarity ratio of the organic compound to the test substance becomes, for example, 1:100 to 20:1, or 1:100 to 10:1 to cause a reaction. The reaction can be caused in the following manner: while the solution including the organic compound and the test substance is kept in a temperature range of, for example, about 4°C to about 60°C, it is stirred or left at stand for ordinarily about 1 minute to about 2 days.

In a case where the molarity of the test substance cannot be adjusted, it may be dissolved in, for example, water, an organic solvent such as methanol, ethanol, acetonitrile, or acetone, or a solvent mixture of the foregoing (a solvent mixture of water and an organic solvent, or a solvent mixture of two or more organic solvents) such that the concentration becomes, for example, about 0.01 mg/mL to about 10 mg/mL, ordinarily about 0.1 mg/L to about 1 mg/mL. Subsequently, a solution of about 0.1 to about 100 μg/mL, ordinarily 1 to 10 μg/mL, of the organic compound serving as a measurement basis of a reagent for determining skin allergenicity may be added, to the test substance solution, in an amount equal to that of the test substance solution. However, as long as the concentration and the amount of addition satisfy such ranges, adjustments may be appropriately performed: for example, the concentration of the measurement reagent is doubled while the amount of the measurement reagent added is halved. The reaction can be performed in the following manner: while the solution including the organic compound and the test substance is kept in a temperature range of, for example, about 4°C to about 60°C, it is stirred or left at stand for ordinarily about 1 minute to about 2 days.

In the reaction, the reactivity between the organic compound and the test substance is determined, to thereby determine the skin allergenicity of the test substance. In order to determine the reactivity, analysis is performed in terms of the residual amount of the organic compound in the solution mixture of the solution of the reagent for determining skin allergenicity and the test substance solution and/or the generation amount of the reaction product of the organic compound and the test substance. This analysis is performed over time, and the reaction rate constant between the organic compound and the test substance is determined and compared with the reaction rate constant of another test substance, or the reaction rate constant of the test substance is compared with the reaction rate constant of a compound that was tested by animal experiments in terms of the presence or absence of and the degree of skin allergenicity. This enables evaluation of the skin allergenicity of the test substance.

Incidentally, in the analysis of the residual amount, when the reagent for determining skin allergenicity can undergo some change in the reaction solution, as needed, reaction solutions (control group) not including only the test substance may be separately prepared and analyzed, and the values of the residual amounts of these reaction solutions may be used for correction.

For example, when the reagent for determining skin allergenicity has a thiol group, it may be easily oxidized into a disulfide (dimer). Thus, in the quantification of the residual amount of the organic compound, as needed, the analysis may also be performed for the disulfide, and the total amount of the organic compound and the disulfide may be calculated as the residual amount of the organic compound.

The reactivity (covalent bondability) between the test substance and the nucleophilic reagent can be estimated by quantification of the unreacted nucleophilic reagent; however, detection and quantification of the product of the reaction (reaction product) between the test substance and the nucleophilic reagent is the most direct and accurate evaluation. In the case of a mixture, when its constituent components are known and the reaction products of the components and the nucleophilic reagent are available, the reaction products can be subjected to, for example, an HPLC-fluorescence method to thereby be quantified.

The method of analyzing the organic compound or the reaction product is not particularly limited, but preferably includes subjecting, to chromatography treatment, a reaction product obtained in the step of causing a reaction between the organic compound and a mixture containing two or more test substances. For example, high-performance liquid chromatography (HPLC), gas chromatography (GC), or thin layer chromatography (TLC) may be employed to isolate and evaluate the compound generated by the reaction, the organic compound, and the test substances. Examples of the chromatographic technique applicable to such HPLC, GC, or TLC include reversed phase chromatography, normal phase chromatography, and ion exchange. Regarding commercially available columns and TLC usable for such chromatographic techniques, examples of LC columns include CAPCELL CORE C18 (manufactured by OSAKA SODA CO., LTD.), CAPCELL-PAK (manufactured by Shiseido Company, Limited), L-column ODS (manufactured by Chemicals Evaluation and Research Institute, Japan), and Shodex Asahipak (manufactured by SHOWA DENKO K. K.); examples of TLC plates include silica gel 60F254 (manufactured by Merck KGaA) and Silica Gel Plate (manufactured by NACALAI TESQUE, INC.).

In the present invention, the amount of organic compound after the reaction between the test substances and the nucleophilic reagent, or the amount of product of the reaction is determined by an optical measurement using a fluorescence detector.

Molecules in the ground state absorb excitation light and undergoes transition to the excitation state. The absorbed excitation energy partially decays as, for example, vibration energy; non-radiative transition to a lower vibration level occurs, and then returning to the ground state occurs to emit light that is fluorescence. In general, the optical measurement using a fluorescence detector is said to be an analytical method that has $10^3$ or more times higher sensitivity than absorption spectrophotometry. In addition, the optical measurement is directed to fluorescent substances, and hence is used as an analytical method that has high selectivity and is used for analyses of very small amounts of substances. The fluorescence intensity is proportional to the concentration of the fluorescent substance, and a calibration curve is created to perform quantitative analysis. The fluorescence detector may be a commercially available detector, and examples include detectors manufactured by SHIMADZU CORPORATION, Waters Corporation, Hitachi, Ltd., Agilent Technologies, or OSAKA SODA CO., LTD.

In the optical measurement using a fluorescence detector, the excitation wavelength is preferably 200 to 350 nm, more preferably 230 to 320 nm, still more preferably 250 to 300 nm, yet more preferably 270 to 300 nm, particularly preferably 280 to 290 nm. The fluorescence wavelength is preferably 200 to 400 nm, more preferably 300 to 370 nm, still more preferably 300 to 360 nm, particularly preferably 320 to 350 nm.

Calculation of depletion percent

From the average values (each, n = 3) of peak areas of the post-reaction unreacted "organic compound that has at least one thiol group and emits fluorescence" or "organic compound that has at least one amino group and emits fluorescence", and the blank-test (after performing the reaction step without addition of the test substances) "organic compound that has at least one thiol group and emits fluorescence" or "organic compound that has at least one amino group and emits fluorescence", the following formula is used to calculate the depletion percent of the "organic compound that has at least one thiol group and emits fluorescence" or the "organic compound that has at least one amino group and emits fluorescence". The phrase of performing the reaction step without addition of the test substances means that, in the step of causing a reaction between the organic compound and the test substances, the reaction step is performed without including the test substances.

Depletion percent (% depletion) of organic compound that has at least one thiol group and emits fluorescence = [1 - (Average value of peak areas of post-reaction unreacted "organic compound that has at least one thiol group and emits fluorescence"/Average value of peak areas of blank-test (after performing the reaction step without addition of the test substances) "organic compound that has at least one thiol group and emits fluorescence")] $\times$ 100

Depletion percent (% depletion) of organic compound that has at least one amino group and emits fluorescence = [1 - (Average value of peak areas of post-reaction unreacted "organic compound that has at least one amino group and emits fluorescence"/Average value of peak areas of blank-test (after performing the reaction step without addition of the test substances) "organic compound that has at least one amino group and emits fluorescence")] $\times$ 100

Determination criteria for prediction of skin allergenicity

In the case of determining the amount of the post-reaction organic compound by an optical measurement using a fluorescence detector, for example, the depletion percent of the organic compound before and after the reaction is calculated, to thereby determine skin allergenicity.

The depletion percent of the "organic compound that has at least one thiol group and emits fluorescence" or the "organic compound that has at least one amino group and emits fluorescence" or the average score of the two depletion percents is calculated, and whether the test substances are "allergenic substances" or "non-allergenic substances" can be determined:

(1) In the case of performing evaluation based on the average value of the depletion percent of the organic compound that has at least one thiol group and emits fluorescence and the depletion percent of the organic compound that has at least one amino group and emits fluorescence,

Average score < 4.9%: the test substances are determined as non-allergenic substances,
Average score $\geq$ 4.9%: the test substances are determined as allergenic substances, or

(2) In the case of performing evaluation based on only the depletion percent of the organic compound that has at least one thiol group and emits fluorescence,

Depletion percent of organic compound that has at least one thiol group and emits fluorescence < 5.6%: the test substances are determined as non-allergenic substances,
Depletion percent of organic compound that has at least one thiol group and emits fluorescence $\geq$ 5.6%: the test substances are determined as allergenic substances.

The present invention will be described further in detail with reference to the following Examples. However, the present invention is not limited to the Examples.

EXAMPLES

Abbreviations in EXAMPLES have the following meanings.

EDTA: ethylenediaminetetraacetic acid
NAC: N-[2-(naphthalen-1-yl)acetyl]cysteine
NAL: $\alpha$-N-[2-(naphthalen-1-yl)acetyl]lysine
TFA: trifluoroacetic acid

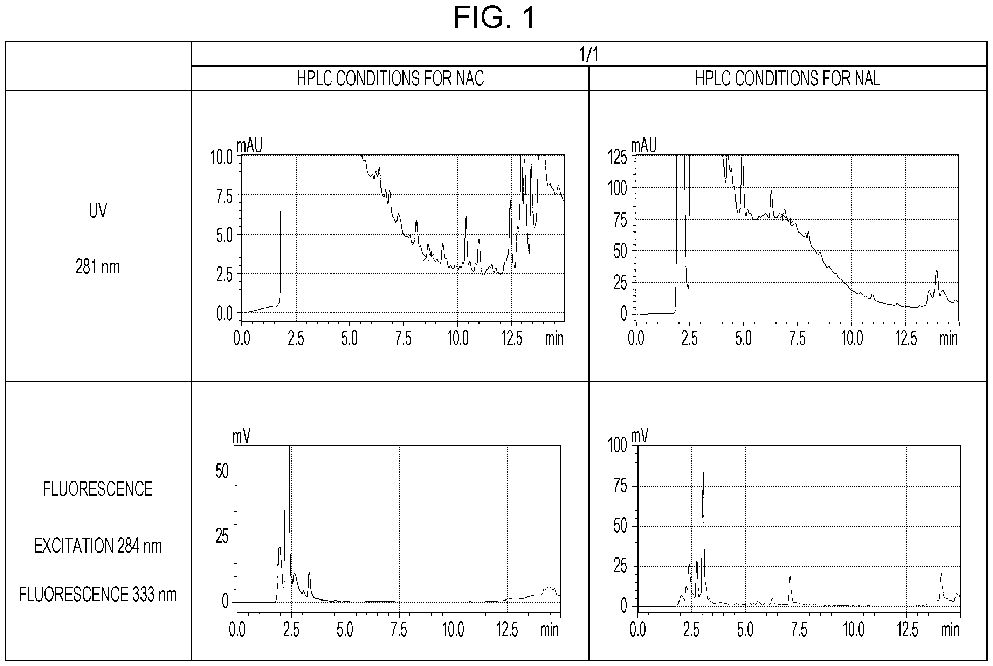

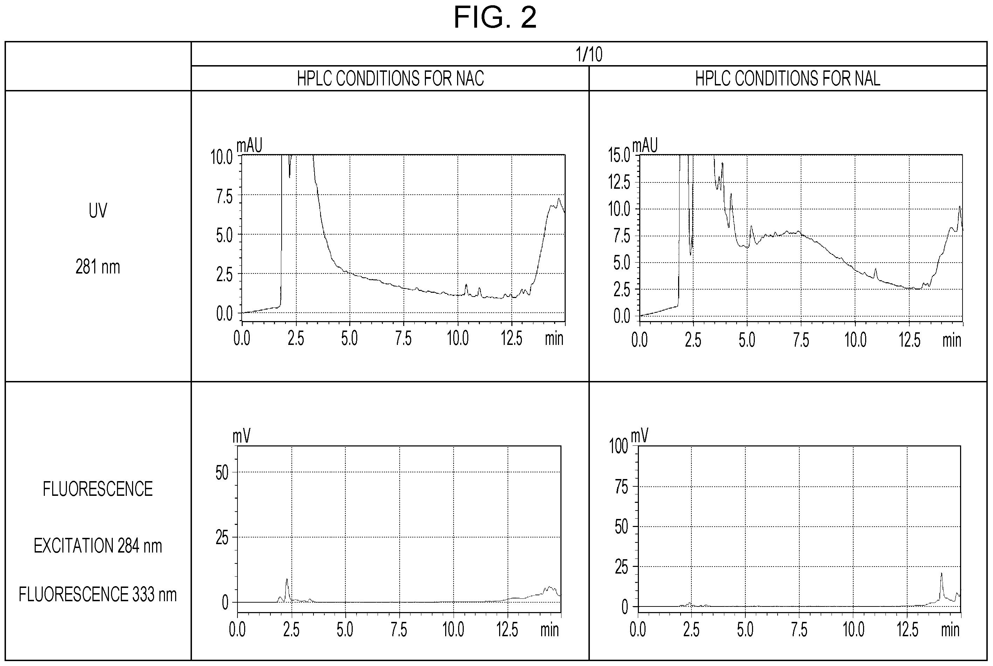

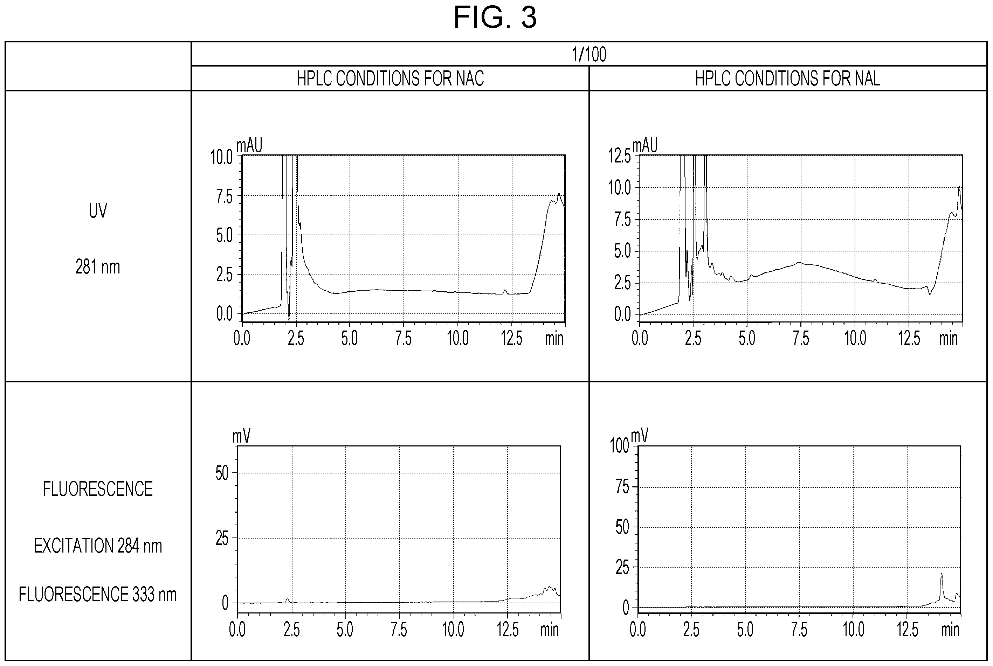

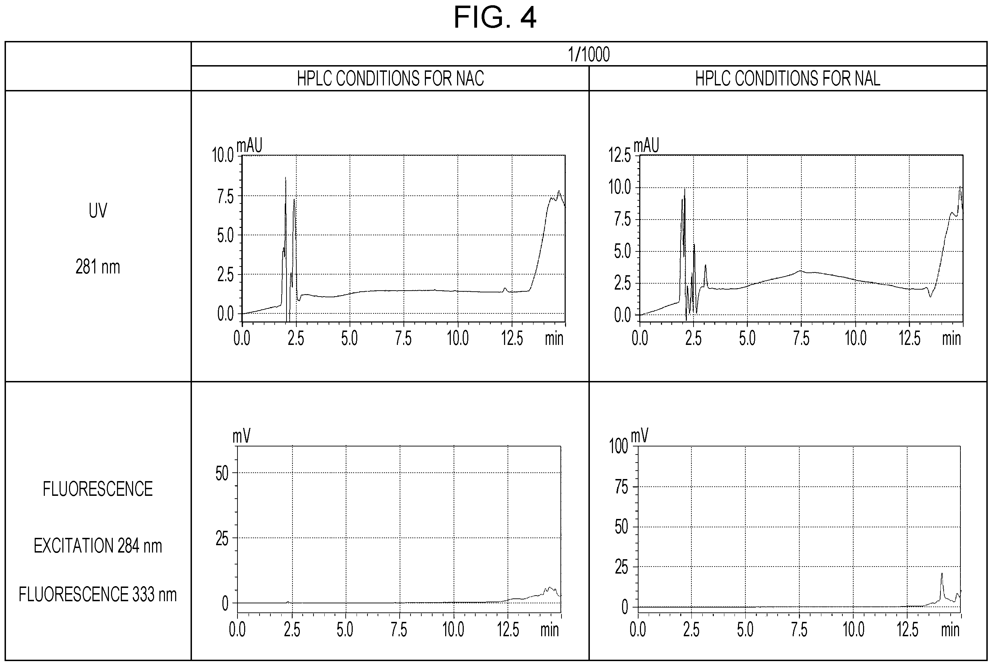

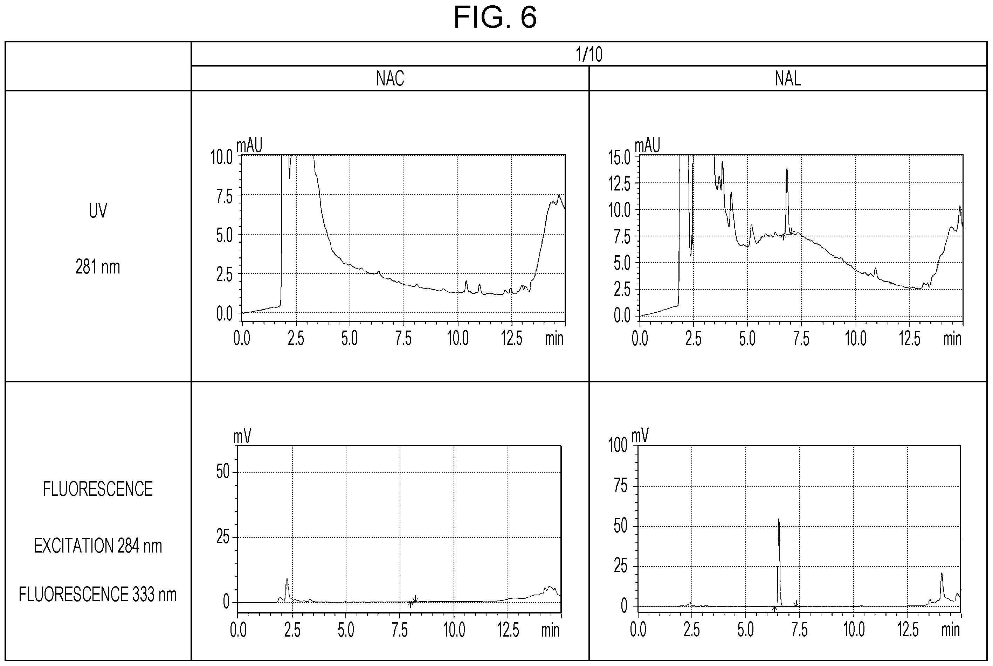

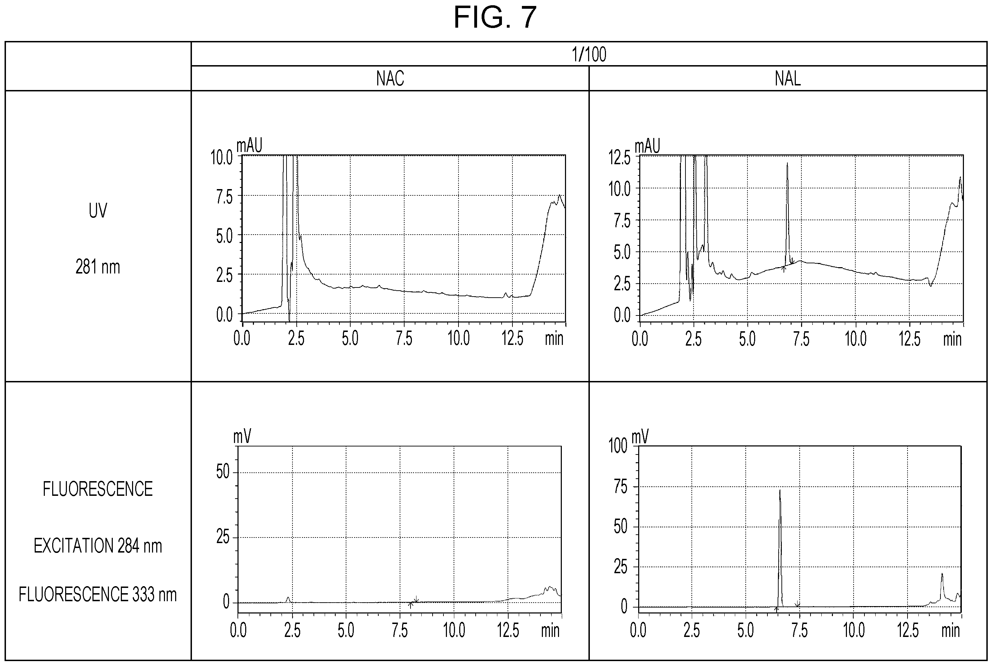

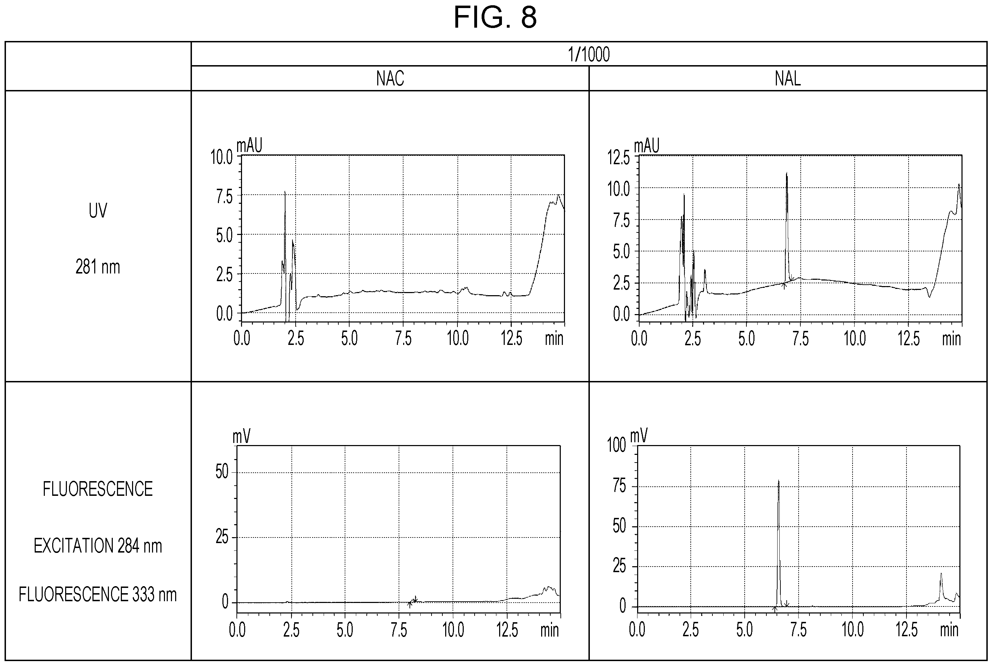

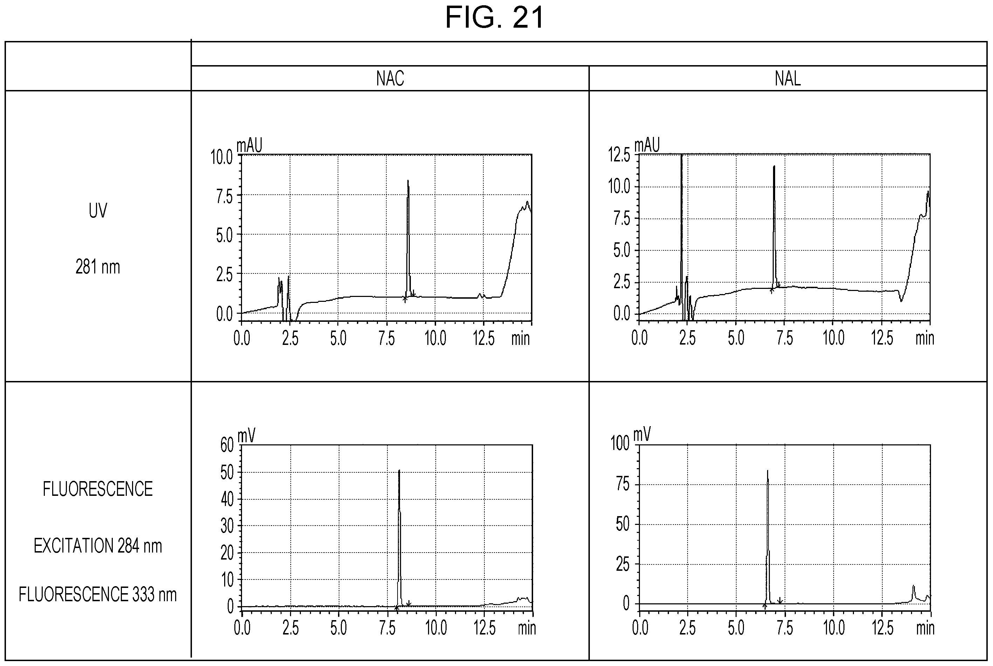

In Fig. 1 to Fig. 21, the abscissa axis indicates retention time (min), and the ordinate axis indicates, in the case of UV (ultraviolet), Absorbance Unit (mAU), or, in the case of fluorescence, voltage (mV).

8

Test method

(1) Preparation of solutions

(1-1) 0.1 mmol/L EDTA aqueous solution

1) To a 15 mL conical tube, 37.2 mg of EDTA·2Na·2H$_2$O (manufactured by DOJINDO LABORATORIES) is weighed and dissolved by using a 25 mL measuring pipet to add 10 mL of distilled water (manufactured by HIKARI PHARMACEUTICAL CO., LTD., water for injection, Japanese Pharmacopoeia) (10 mmol/L EDTA aqueous solution).

2) To a 100 mL vessel, 49.5 mL of distilled water (manufactured by HIKARI PHARMACEUTICAL CO., LTD., water for injection, Japanese Pharmacopoeia) is added using a 50 mL measuring pipet; to this, 0.5 ml of the 10 mmol/L EDTA aqueous solution of 1) above is added and mixed to achieve 100-fold dilution (0.1 mmol/L EDTA aqueous solution).

(1-2) 100 mmol/L phosphate buffer (pH: 8.0)

1) To a 100 mL vessel, 0.6 g of anhydrous sodium dihydrogenphosphate (manufactured by FUJIFILM Wako Pure Chemical Corporation, Special Grade) is weighed and dissolved by using a 50 mL measuring pipet to add 50 mL of distilled water ((manufactured by HIKARI PHARMACEUTICAL CO., LTD., water for injection, Japanese Pharmacopoeia)).

2) To a 500 mL vessel, a 50 mL (or 100 mL) measuring pipet is used to add 300 mL of distilled water (manufactured by HIKARI PHARMACEUTICAL CO., LTD., water for injection, Japanese Pharmacopoeia).

3) Anhydrous disodium hydrogenphosphate (manufactured by FUJIFILM Wako Pure Chemical Corporation, Special Grade, 4.26 g) is weighed and dissolved by being added to the distilled water (manufactured by HIKARI PHARMACEUTICAL CO., LTD., water for injection, Japanese Pharmacopoeia) of 2).

4) To the anhydrous disodium hydrogenphosphate solution of 3), 16 mL of the anhydrous sodium dihydrogenphosphate solution of 1) is added using a 25 mL measuring pipet.

5) A 25 mL measuring pipet is used to remove 17 mL of the solution of 4); to the remaining solution of 4), 1 mL of a 0.1 mmol/L EDTA aqueous solution is added to provide a 300 mL solution. This solution has an EDTA concentration of 0.33 μmol/L, and the concentration becomes 0.25 μmol/L in the reaction solution.

6) An aliquot of the solution is taken to another vessel, and measured for pH using a pH meter, to confirm that the pH is in the range of 7.9 to 8.1.

(1-3) 100 mmol/L phosphate buffer (pH: 10.2)

1) A 50 mL measuring pipet is used to add, to a 500 mL vessel, 286 mL of distilled water (manufactured by HIKARI PHARMACEUTICAL CO., LTD., water for injection, Japanese Pharmacopoeia).

2) Anhydrous disodium hydrogenphosphate (4.26 g) is weighed, and dissolved by being added to the distilled water (manufactured by HIKARI PHARMACEUTICAL CO., LTD., water for injection, Japanese Pharmacopoeia) of 1).

3) A 0.1 mol/L NaOH aqueous solution (manufactured by FUJIFILM Wako Pure Chemical Corporation, Special Grade, 14 mL) is added using a 25 mL measuring pipet.

4) An aliquot of the solution is taken to another vessel, and measured with a pH meter (portable pH meter (HM-20P), TOA Electronics Ltd.) for pH, to confirm that the pH is in a range of 10.1 to 10.3.

(1-4) Reaction stopping solution (2.5% (v/v) TFA aqueous solution)

To 100 mL of distilled water (manufactured by FUJIFILM Wako Pure Chemical Corporation), 2.5 mL of TFA (manufactured by FUJIFILM Wako Pure Chemical Corporation, Special Grade) is added.

(1-5) HPLC mobile phase A: 0.1% (v/v) TFA aqueous solution

To 1 L of distilled water (manufactured by FUJIFILM Wako Pure Chemical Corporation), 1.0 mL of TFA is added.

(1-6) HPLC mobile phase B: 0.1% (v/v) TFA acetonitrile solution

To 1 L of HPLC-grade acetonitrile (manufactured by FUJIFILM Wako Pure Chemical Corporation, for HPLC), 1.0 mL of TFA is added.

(2) Preparation of nucleophilic reagent stock solutions

(2-1) Preparation of NAC stock solution

Within each test, the same stock solution is used. The stock solution is stored in portions having an amount that can be consumed in each test. A specific preparation example is as follows.

1) To a 50 mL vessel, 11.6 mg ($\pm$0.1 mg) of NAC is weighed; to this, a 25 mL measuring pipet is used to add 20 mL of 100 mmol/L phosphate buffer (pH: 8.0); and the content is gently stirred using a test tube mixer to achieve dissolution (2 mmol/L).
2) To a 500 mL vessel, 149.5 mL of this buffer is added using a 50 mL measuring pipet; to this, 0.5 mL of the above-described 2 mmol/L NAC solution is added; and the content is mixed by inversion to achieve 300-fold dilution (6.667 $\mu$mol/L).

(2-2) Preparation ofNAL stock solution (molecular weight: 314.38)

Within each test, the same stock solution is used. The stock solution is stored in portions having an amount that can be consumed in each test. A specific preparation example is as follows.

1) To a 50 mL vessel, 12.6 mg ($\pm$0.1 mg) of NAL is weighed; to this, a 25 mL measuring pipet is used to add 20 mL of 100 mmol/L phosphate buffer (pH: 10.2); and the content is gently stirred using a test tube mixture to achieve dissolution (2 mmol/L NAL solution).
2) To a 500 mL vessel, 149.5 mL of this buffer is added using a 50 mL measuring pipet; to this, 0.5 mL of the above-described 2 mmol/L NAL solution is added; and the content is mixed by inversion to achieve 300-fold dilution (6.667 $\mu$mol/L).

(3) Preparation of test substance (multi-component mixture) solution

(3-1) Case where test substance is liquid

The test substance undiluted solution is defined as an "undiluted solution", and used. Incidentally, this "undiluted solution" may be appropriately diluted to, for example, 1/10, 1/100, or 1/1000, using an appropriate solvent, and the resultant solution may be used.

(3-2) Case where test substance is solid

The test substance is dissolved, to reach the maximum dissolution concentration, in a solvent in which the test substance exhibits the highest solubility. Subsequently, the solution having the maximum dissolution concentration is defined as an "undiluted solution", and tested. Incidentally, this "undiluted solution" may be appropriately diluted to, for example, 1/10, 1/100, or 1/1000, using an appropriate solvent, and the resultant solution may be used.

(4) Preparation of test substance (single substance) solution

The test substance is prepared at 1 mmol/L in any of solvents that are water, acetonitrile, acetone, and a 5 mass% dimethyl sulfoxide (DMSO)/acetonitrile solution and used as a test substance solution. As the solvent, a solvent in which the test substance dissolves at 1 mmol/L is used; when the test substance is soluble in a plurality of solvents, the solvent is selected in the following order of decreasing precedence: water, acetonitrile, acetone, and 5 mass% DMSO/acetonitrile.

(5) Reaction

(5-1) Addition

Test substance solutions are prepared on a 96-well plate (U96 PP-0.5 ML NATURAL, Thermo Fisher Scientific Inc. (NUNC)) mainly using a 12-channel pipette, and a reagent is added in accordance with the following formula.

Nucleophilic reagent (NAC and NAL): 150 $\mu$L
Test substance solution: 50 $\mu$L

(5-2) Reaction

The plate is tightly sealed using a plate sealing (Resistant embossed seal, SHIMADZU GLC Ltd.), and stirred using a plate shaker (Titramax 100, Heidolph Instruments GmbH & CO. KG). The plate is subjected to spinning down using a centrifuge, and then to incubation at 25°C, for 24 hours, in a light-shielded state.

(5-3) Stopping of reaction

After the incubation for 24 hours, the plate sealing is removed; to each sample, 50 $\mu$L of the reaction stopping solution is added to stop the reaction.

(6) HPLC measurement

The HPLC measurement conditions for the nucleophilic reagents are as follows.

Table 1

| Table 1: HPLC measurement conditions | |
|---|---|
| HPLC apparatus | LC-20A (Prominence) series (SHIMADZU CORPORATION) |
| Column | CAPCELL CORE C18 column (3.0 $\times$ 150 mm, 2.7 $\mu$m) (OSAKA SODA CO., LTD.) |
| Detectors | UV detection: SPD-M20A (SHIMADZU CORPORATION) |
| | Fluorescence detection: RF10AXL (SHIMADZU CORPORATION) |
| Detection wavelengths | UV detection: 281 nm |
| | Fluorescence detection: 284 nm (excitation), 333 nm (fluorescence) |
| Column temperature | 40°C |
| Sample temperature | 25°C |
| Injection amount | 10-20 $\mu$L |
| Eluants | A: water (0.1% trifluoroacetic acid) |
| | B: acetonitrile (0.1% trifluoroacetic acid) |
| Measurement time | 20 minutes |

(continued)

| Table 1: HPLC measurement conditions | | | | |
|---|---|---|---|---|
| Elution conditions | **NAC** | | | |
| | Time (min) | Flow rate (ml/min) | %A | %B |
| | 0.0 | 0.3 | 70 | 30 |
| | 9.5 | 0.3 | 45 | 55 |
| | 10.0 | 0.3 | 0 | 100 |
| | 13.0 | 0.3 | 0 | 100 |
| | 13.5 | 0.3 | 70 | 30 |
| | 20.0 | End | | |
| | **NAL** | | | |
| | Time (min) | Flow rate (mL/min) | %A | %B |
| | 0.0 | 0.3 | 80 | 20 |
| | 9.5 | 0.3 | 55 | 45 |
| | 10.0 | 0.3 | 0 | 100 |
| | 13.0 | 0.3 | 0 | 100 |
| | 13.5 | 0.3 | 80 | 20 |
| | 20.0 | End | | |

(7) Data analysis

(7-1) Calculation of depletion percent

From the average values (each, n = 3) of peak areas of the post-reaction unreacted NAC or NAL and the blank-test (after performing the reaction step without addition of the test substances) NAC or NAL, the following formula is used to calculate the depletion percent of NAC or NAL.

NAC depletion percent (% depletion) = [1 - (Average value of peak areas of post-reaction unreacted NAC/Average value of peak areas of blank-test (after performing the reaction step without addition of the test substances)NAC)] $\times$ 100

NAL depletion percent (% depletion) = [1 - (Average value of peak areas of post-reaction unreacted NAC/Average value of peak areas of blank-test (after performing the reaction step without addition of the test substances)NAL)] $\times$ 100

The average value of peak areas of the post-reaction unreacted NAC is the average value of areas obtained by measuring three times post-reaction unreacted NAC. The average value of peak areas of blank-test (after performing the reaction step without addition of the test substances) NAC is the average value of areas obtained by measuring three times blank-test (after performing the reaction step without addition of the test substances) NAC.

The average value of peak areas of post-reaction unreacted NAL is the average value of areas obtained by measuring three times post-reaction unreacted NAL. The average value of peak areas of blank-test (after performing the reaction step without addition of the test substances) NAL is the average value of areas obtained by measuring three times blank-test (after performing the reaction step without addition of the test substances) NAL.

(7-2) Average score

The average value (Average Score) of NAC and NAL depletion percents is calculated. In the resultant value, the second decimal place is rounded off.

Average value (Average Score) of NAC and NAL depletion percents = (NAC depletion percent + NAL depletion percent)/2

(8) Prediction of skin allergenicity

Determination criteria:

The depletion percent of at least one of NAC or NAL or the average score of the depletion percents of NAC and NAL is calculated, and whether or not the test substances are "allergenic substances" or "non-allergenic substances" is determined:

(1) in a case of performing evaluation based on the average value of the depletion percent of NAC and the depletion percent of NAL,

Average score < 4.9%: the test substances are determined as non-allergenic substances,
Average score $\geq$ 4.9%: the test substances are determined as allergenic substances, or (2) in a case of performing evaluation based on only the depletion percent of NAC,
Depletion percent of NAC < 5.6%: the test substances are determined as non-allergenic substances,
Depletion percent of NAC $\geq$ 5.6%: the test substances are determined as allergenic substances.

Example 1: Prediction of skin allergenicity of green-tea extract

A commercially available green-tea extract was obtained. This extract was diluted, with acetonitrile, to 1/10, 1/100, and 1/1000. The green-tea extract undiluted solution and diluted solutions were subjected to prediction of skin allergenicity by a fluorescence method.

Mixture including test substances

A green-tea extract (green-tea liquid obtained from ICHIMARU PHARCOS Co., Ltd.) was used. The extraction solvent has water:ethanol:1,3-butylene glycol = 2: 1: 1. The result of an in vivo skin allergenicity test of this green-tea liquid is positive (skin allergenic substances). This extract undiluted solution and solutions prepared by diluting this extract undiluted solution, using acetonitrile, to 1/10, 1/100, and 1/1000 were used in experiments.

Experimental conditions

The above-described green-tea extract undiluted solution and diluted solutions were individually caused to react, at 25°C for 24 hours, with NAC or NAL serving as a nucleophilic reagent. The reactions were each performed on a 96-well plate, and the number of samples of each case was set to n = 3. In addition, as controls, reaction solutions including the solvent alone were prepared. The reaction solutions were prepared so as to have a NAC or NAL concentration of 5 $\mu$mol/L. To the reaction solutions after the lapse of 24 hours, a trifluoroacetic acid (TFA) aqueous solution was added such that the final concentration became 0.5% (v/v) TFA. Subsequently, HPLC was performed for measurements by two detection methods that were light absorption detection using a photodiode array (PDA) detector, and fluorescence detection using a fluorescence detector. The resultant chromatographs and depletion percents (Depletion) of NAC and NAL were compared.

Measurement conditions

Under the HPLC measurement conditions described in Table 1 above, depletion (%) of the nucleophilic reagents (NAC and NAL) was determined.

Results

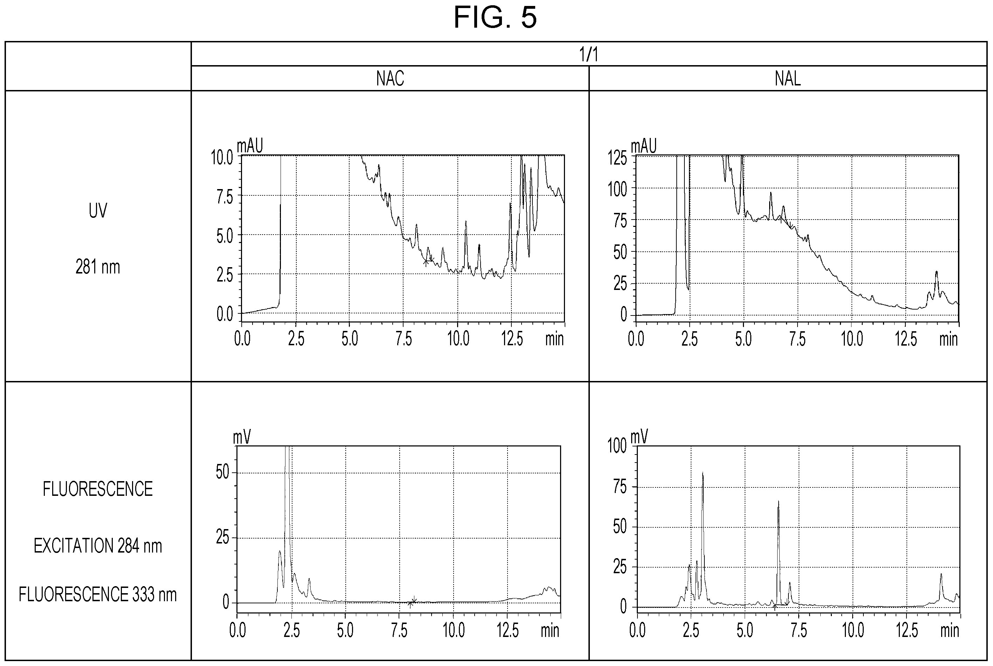

The green-tea extract and the diluted solutions were subjected to HPLC measurements by UV detection and fluorescence detection. Fig. 1 to Fig. 4 include HPLC charts by UV detection and fluorescence detection for the green-tea extract and its diluted solutions alone (not including any nucleophilic reagent). Fig. 5 to Fig. 8 include HPLC charts by UV detection and fluorescence detection for the reaction solutions of the green-tea extract or its diluted solutions and the nucleophilic reagent (NAC or NAL).

Discussion

As a result of the HPLC measurement of the green-tea extract undiluted solution, in the detection of UV at 281 nm, a large number of peaks of multiple components were observed and hence both of the nucleophilic reagents (NAC and NAL) were not detected or quantified. For the 1/10 diluted solution of the green-tea extract, the intensities of the peaks of the multiple components lowered, but did not lower to the baselines at the peaks of the nucleophilic reagents, so that accurate quantification was difficult. For the 1/100 and 1/1000 diluted solutions, the lowering to the baselines occurred at the positions of the nucleophilic reagents to such an extent that the quantification was not affected, so that the nucleophilic reagents were quantified.

By contrast, when the green-tea component undiluted solutions were subjected to fluorescent detection, most of the multiple components did not emit fluorescence, and hence the baselines were very low and stable. Thus, only the peaks of the nucleophilic reagents (NAC and NAL) were detected, so that accurate quantification was achieved without being affected by the multiple components. However, NAC reacts with green-tea extract components, so that peaks were not detected after the reaction for 24 hours. Similarly, for the 1/10, 1/100, and 1/1000 diluted solutions of the green-tea extract, the nucleophilic reagents were quantified.

Prediction of skin allergenicity

From the HPLC charts in Fig. 1 to Fig. 8, the depletion percents (Depletion) of the nucleophilic reagents were calculated, and the average scores (Mean Depletion) were determined. On the basis of these values, skin allergenicity was predicted and the results are as follows. In Tables of EXAMPLES, SD represents standard deviation.

Table 2

| | 1/1 | | | | | 1/10 | | | | |
|---|---|---|---|---|---|---|---|---|---|---|
| | NAC | | NAL | | Mean Depletion (%) | NAC | | NAL | | Mean Depletion (%) |
| | Depletion (%) | SD | Depletion (%) | SD | | Depletion (%) | SD | Depletion (%) | SD | |
| UV | Unmeasurable | - | Unmeasurable | - | Unmeasurable | Unmeasurable | 0.0 | Unmeasurable | - | Unmeasurable |
| Fluorescence method | 100.0 | 0.0 | 19.7 | 0.8 | 59.8 | 100.0 | 0.0 | 30.6 | 0.1 | 65.3 |

| | 1/100 | | | | | 1/1000 | | | | |
| --- | --- | --- | --- | --- | --- | --- | --- | --- | --- | --- |
| | NAC | | NAL | | Mean Depletion (%) | NAC | | NAL | | Mean Depletion (%) |
| | Depletion (%) | SD | Depletion (%) | SD | | Depletion (%) | SD | Depletion (%) | SD | |
| UV | 100.0 | 0.0 | 8.7 | 0.0 | 54.4 | 100.0 | 0.0 | 1.5 | 0.2 | 50.8 |
| Fluorescence method | 100.0 | 0.0 | 8.2 | 0.2 | 54.1 | 98.3 | 0.0 | 1.3 | 0.1 | 49.8 |

As is understood from the HPLC charts, the green-tea extract undiluted solution could not be evaluated by UV detection. Similarly, the 1/10 diluted solution could not be evaluated. However, for the 1/100 and 1/1000 diluted solutions, depletion of NAC and NAL was calculated and the average scores were determined. All the average scores were found to be about 50%, which are more than 4.9% defined in the criteria and results in determination of being skin allergenic.

On the other hand, in the fluorescence detection, even for the green-tea extract undiluted solution, the nucleophilic reagents were quantified, and the average score was calculated. Similarly, also for the 1/10, 1/100, and 1/1000 diluted solutions, the depletion and average scores of the nucleophilic reagents were calculated. All the average scores were found to be about 60% to about 50%. Thus, the green-tea extract undiluted solution to the 1/1000 diluted solution were determined as having skin allergenicity.

Discussion

Among the green-tea extracts used, for the undiluted solution and the 1/10 diluted solution, quantification was not achieved by the ordinary HPLC-UV method, but accurate quantification was achieved by the HPLC-fluorescence method of detecting the fluorescence of the nucleophilic reagents without being hindered by the green-tea components, and both solutions were found to inferentially have skin allergenicity.

The 1/100 and 1/1000 diluted solutions in which quantification was achieved by HPLC-UV were respectively found to have average scores of 54.4% and 50.8%, and were found to inferentially have skin allergenicity. Similarly, the average scores of the 1/100 and 1/1000 diluted solutions calculated on the basis of fluorescence detection were respectively found to be 54.1% and 49.8%, which demonstrated that skin allergenicity is inferred as in the UV detection method. Comparison between the average scores obtained by the UV method and the fluorescence method has revealed that the scores were perfectly matched except for minor errors as described above. This has demonstrated that the fluorescence detection method provides measurement at the same accuracy as in the UV detection method. In addition, these results matched the results that the green-tea extract used was determined as being a skin allergenic substance in the in vivo skin allergenicity test. Thus, the results have demonstrated that the fluorescence detection method according to the present invention enables skin-allergenicity evaluation of even a mixture including two or more test substances.

Example 2: Prediction of skin allergenicity of aloe model extract (solution mixture of 20 components)

As main components of an aloe extract, there are 20 or more known components described in a document (D. T. Loots, F. H. van der Westhuizen, and L. Botes, 2007, Aloe ferox leaf gel phytochemical content, antioxidant capacity, and possible health benefits, Journal of Agricultural and Food Chemistry, 55: 6891-6896). These individual components and a model extract as a mixture of all 20 components were prepared and subjected to prediction of skin allergenicity by an UV method and a fluorescence method.

Test substances

Regarding 20 main components of the aloe extract, their names, concentrations, and solvents are described in Table below. Solutions of individual components and 20 components were prepared by the following method.

(1) Method of preparing solutions of individual components

The components were dissolved in solvents described below to prepare stock solutions; the stock solutions were added so as to satisfy the final concentrations, and made up to volume with ethanol.

Solvents

Basically, ethanol (EtOH) was used. However, components for which two solvents are described in "Solvent" were dissolved only in the solvent described before EtOH. For example, as the solvents of β-sitosterol, 3.21% IPA/EtOH are described; however, the preceding solvent, IPA (isopropanol), was used as the solvent.

Stock solutions

Basically, 50 mg/mL solutions were prepared. However, for xanthine, a 1 mg/mL solution was prepared, and, for thymine, a 100 mg/mL solution was prepared.

Amounts of addition

Appropriate amounts of solutions were taken from the stock solutions, and made up to volume with ethanol to prepare solutions having the final concentrations. For example, β-sitosterol was dissolved in isopropanol to prepare a 50 mg/mL solution; 32.1 µL of this solution was taken and diluted to 1000 µL with ethanol.

(2) Method of preparing solution mixture

Appropriate amounts of solutions were taken from the stock solutions of 20 components as described above, and made up to volume with ethanol, to prepare a solution mixture in which the components individually had the final concentrations.

Table 3

| Source plant | Name of component | Concentration | Solvent |
|---|---|---|---|
| Aloe | β-Sitosterol | 1.605 mg/mL | 3.21% IPA/EtOH |
| | Xanthine | 0.027 mg/mL | 2.66% DMSO/EtOH |
| | Benzoic acid | 0.880 mg/mL | EtOH |
| | p-Toluic acid | 0.840 mg/mL | EtOH |
| | Uracil | 0.700 mg/mL | 1.4% DMSO/EtOH |
| | p-Coumaric acid | 0.455 mg/mL | EtOH |
| | Thymine | 0.430 mg/mL | 0.43% DMSO/EtOH |
| | 3-Methylglutaric acid | 0.385 mg/mL | EtOH |
| | 2-Methyl- 1,3 -prop anediol | 0.355 mg/mL | EtOH |
| | Glycerol | 0.345 mg/mL | EtOH |
| | 2,3-Butanediol | 0.340 mg/mL | EtOH |
| | p-Salicylic acid | 0.190 mg/mL | EtOH |
| | Benzyl alcohol | 0.165 mg/mL | EtOH |
| | 1-Propanol | 0.160 mg/mL | EtOH |
| | Protocatechuic acid | 0.160 mg/mL | EtOH |
| | Isovaleric acid | 0.150 mg/mL | EtOH |
| | Lactic acid (L-lactic acid) | 0.150 mg/mL | EtOH |
| | 2, 6-Dimethyl-4-heptanone | 0.130 mg/mL | EtOH |
| | 4-Hydroxyphenylacetic acid | 0.115 mg/mL | EtOH |
| | Linoleic acid | 0.105 mg/mL | EtOH |
| IPA: isopropanol, DMSO: dimethyl sulfoxide, EtOH: ethanol | | | |

Experimental conditions

The individual components and the solution mixture were individually caused to react, at 25°C for 24 hours, with NAC or NAL serving as a nucleophilic reagent. The reactions were each performed on a 96-well plate; the number of samples of each case was set to n = 3. In addition, as controls, reaction solutions including solvents alone were prepared. The reaction solutions were prepared so as to have NAC and NAL concentrations of 5 µmol/L; the plant components were added, with reference to the concentrations of the extract described in the above-described document (D. T. Loots et al.), for two concentrations that were each concentration described in Table above and the concentration of the 1/10 diluted solution, in a 1/4 amount of the reaction solution (final concentrations: 1/4 and 1/40). To such a reaction solution after the lapse of 24 hours, a trifluoroacetic acid (TFA) aqueous solution was added such that the final concentration became 0.5% (v/v) TFA. Subsequently, HPLC was performed for measurements by two measurement methods that were light absorption detection using a photodiode array (PDA) detector, and fluorescence detection using a fluorescence detector. The resultant chromatographs and depletion percents (Depletion) of NAC and NAL of the two methods were compared.

Measurement conditions

Under the HPLC measurement conditions described in Table 1 above, depletion (%) of the nucleophilic reagents (NAC and NAL) was determined.

Results

The 20-component solution mixture and individual components were subjected to HPLC measurement by UV detection and fluorescence detection.

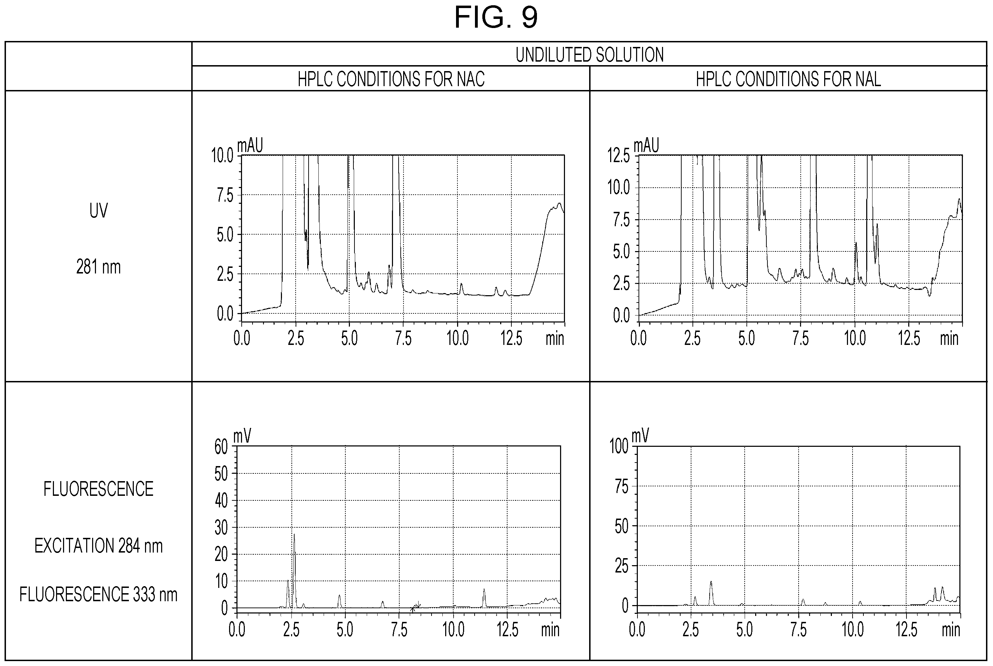

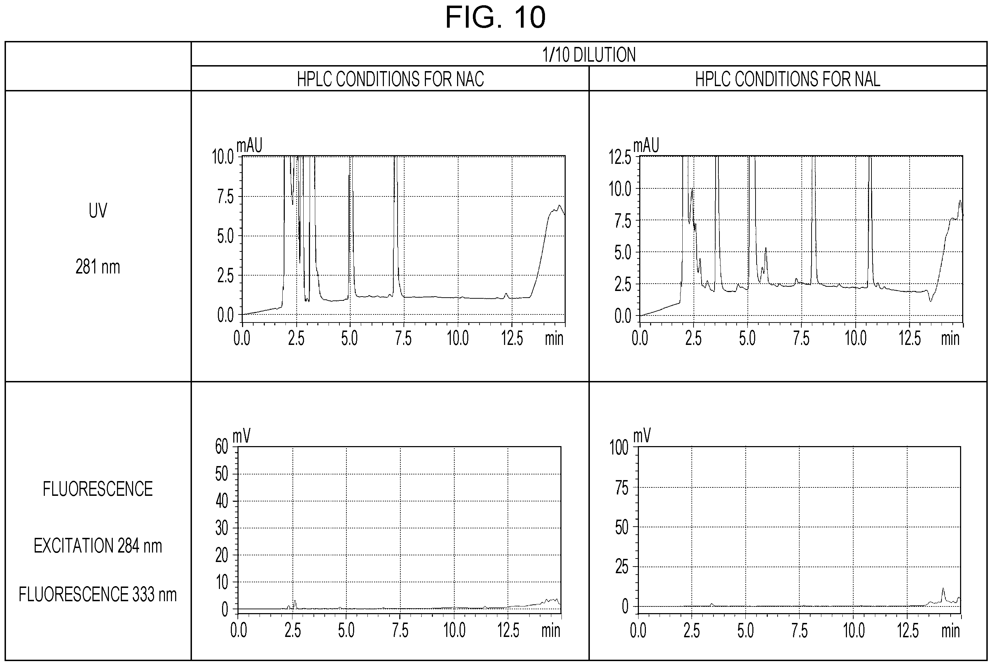

Fig. 9 and Fig. 10 include HPLC charts by UV detection and fluorescence detection for the 20-component solution mixture or its diluted solution alone (not including any nucleophilic reagent).

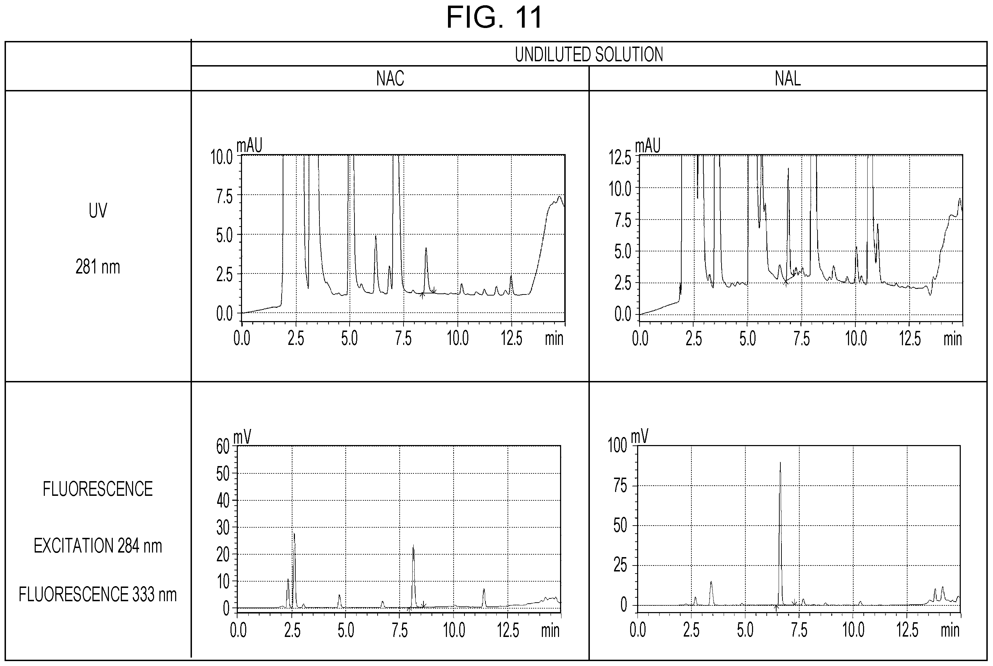

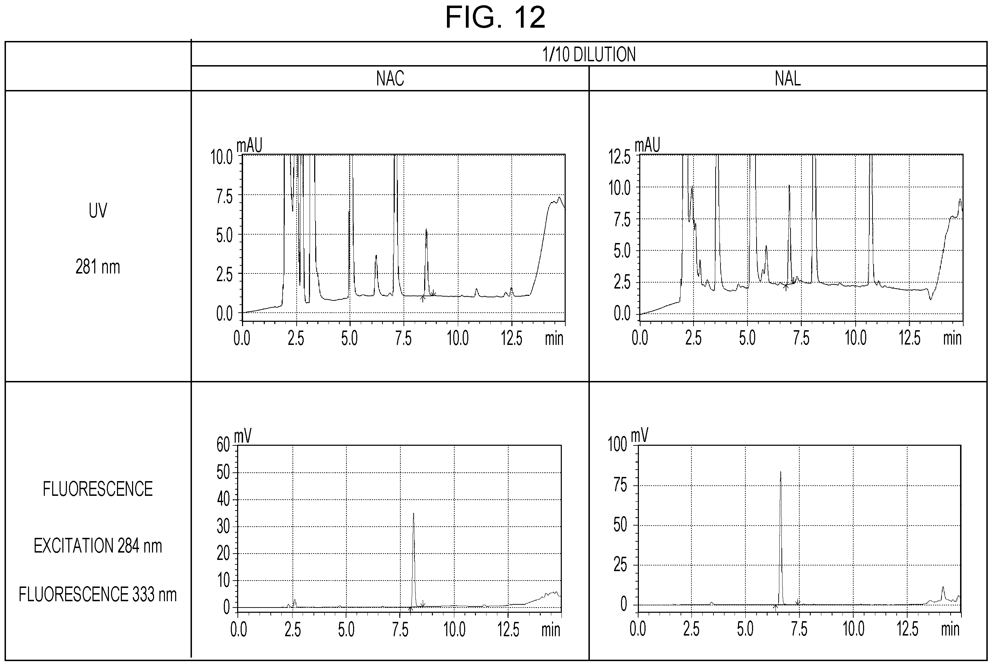

Fig. 11 and Fig. 12 include HPLC charts by UV detection and fluorescence detection for the reaction solution of the 20-component solution mixture or its diluted solution and a nucleophilic reagent (NAC or NAL).

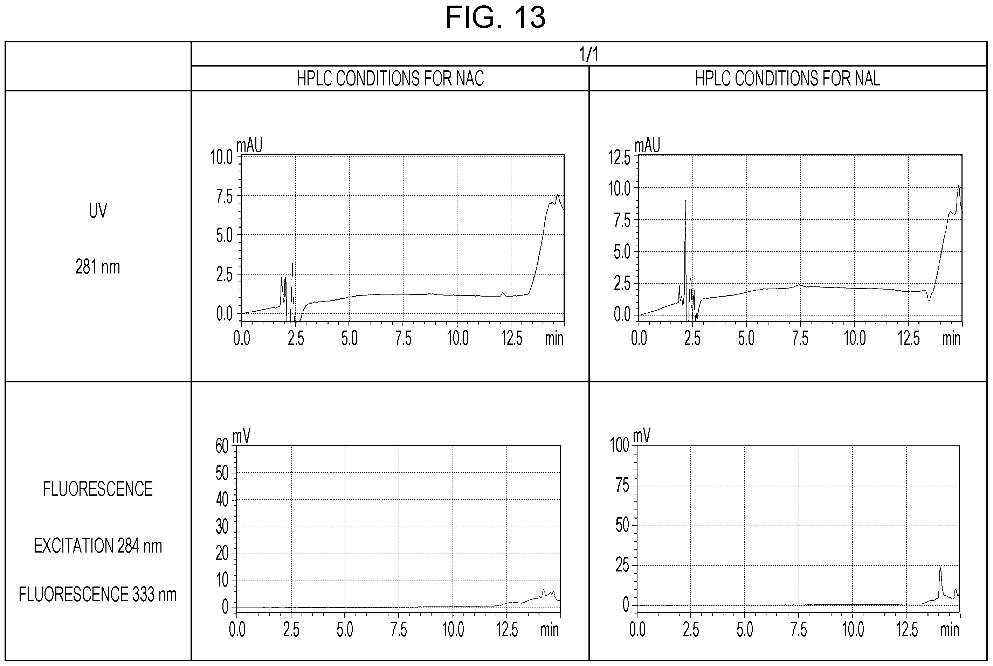

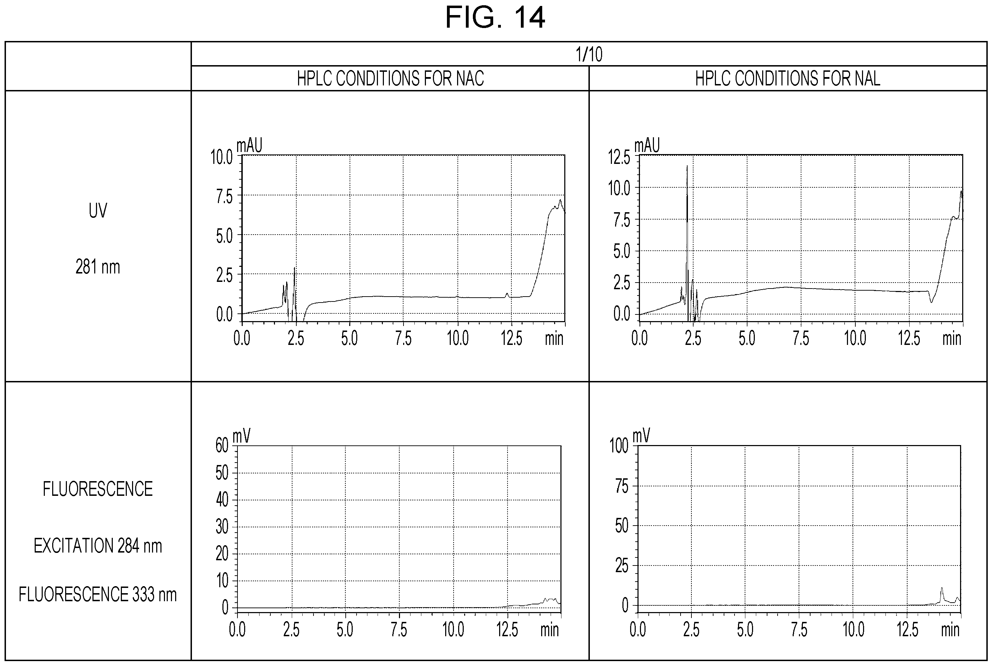

Fig. 13 and Fig. 14 include HPLC charts by UV detection and fluorescence detection for β-sitosterol or its diluted solution alone (not including any nucleophilic reagent).

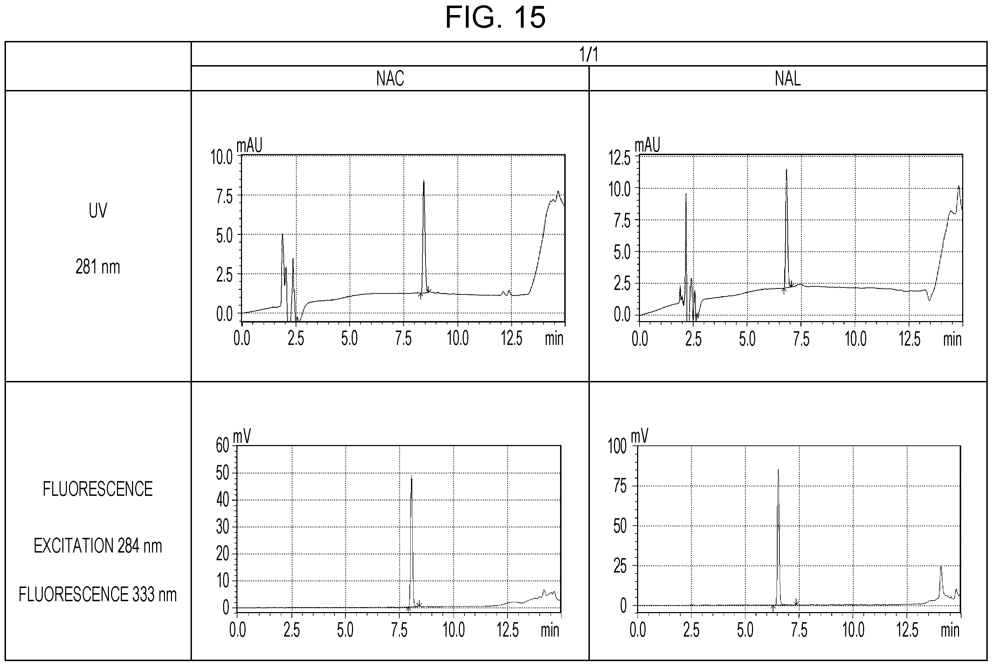

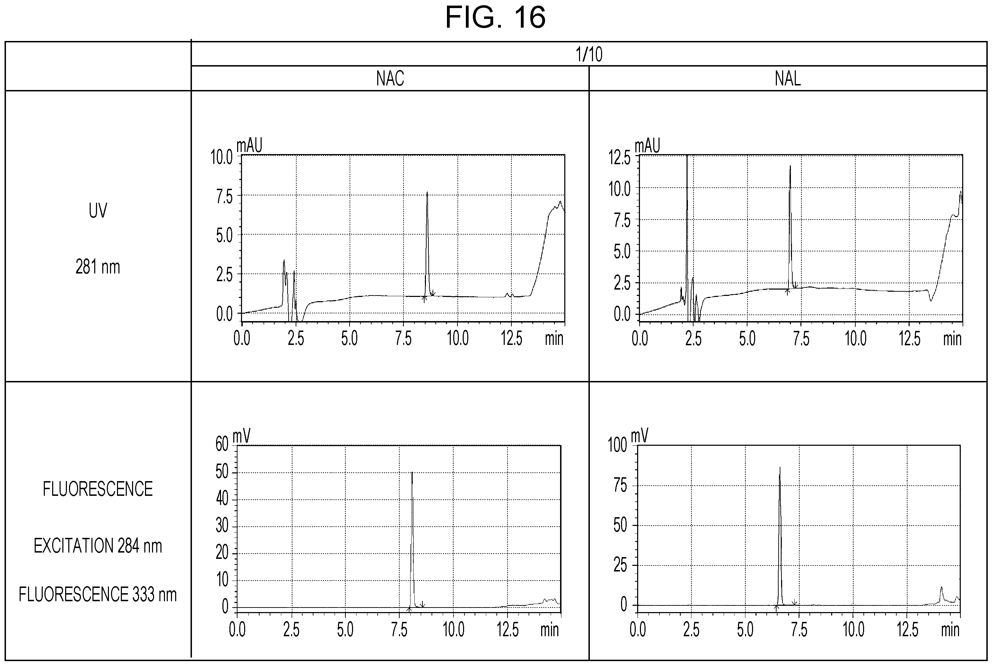

Fig. 15 and Fig. 16 include HPLC charts by UV detection and fluorescence detection for the reaction solution of β-sitosterol or its diluted solution and a nucleophilic reagent (NAC or NAL).

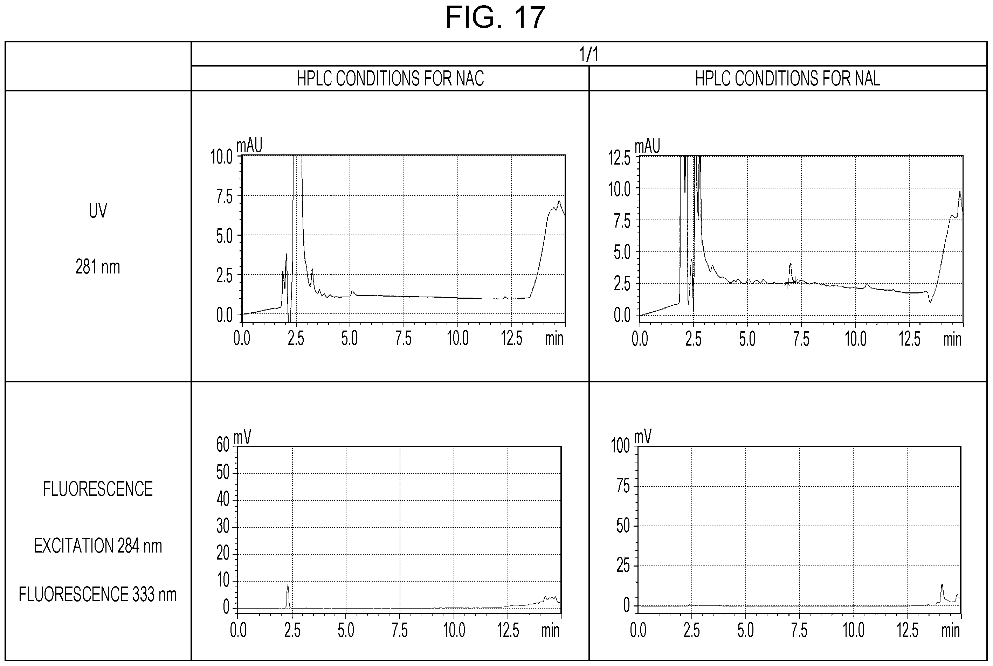

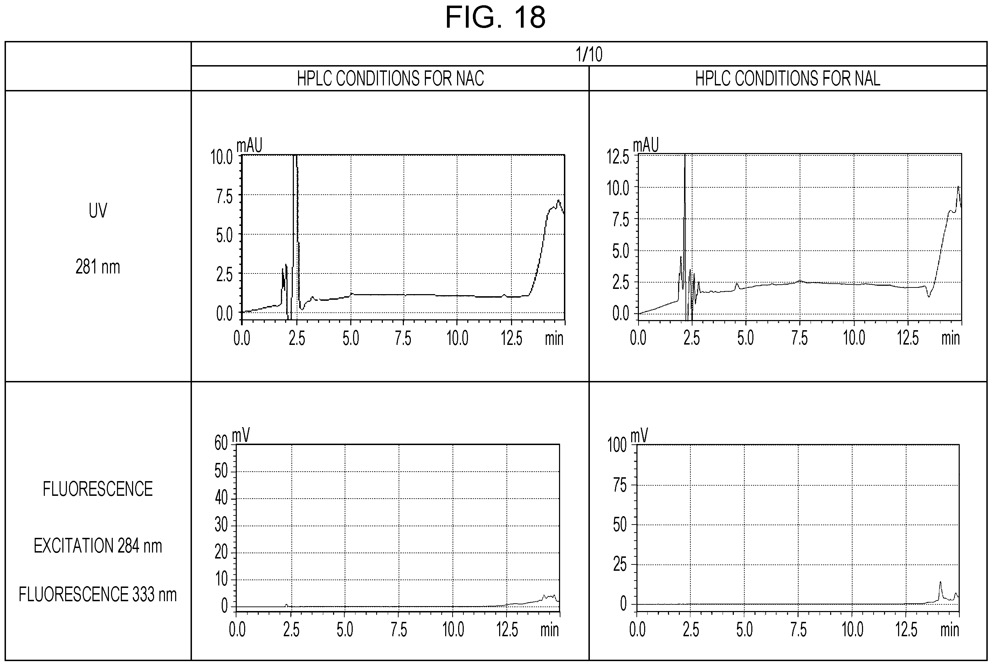

Fig. 17 and Fig. 18 include HPLC charts by UV detection and fluorescence detection for protocatechuic acid or its diluted solution alone (not including any nucleophilic reagent).

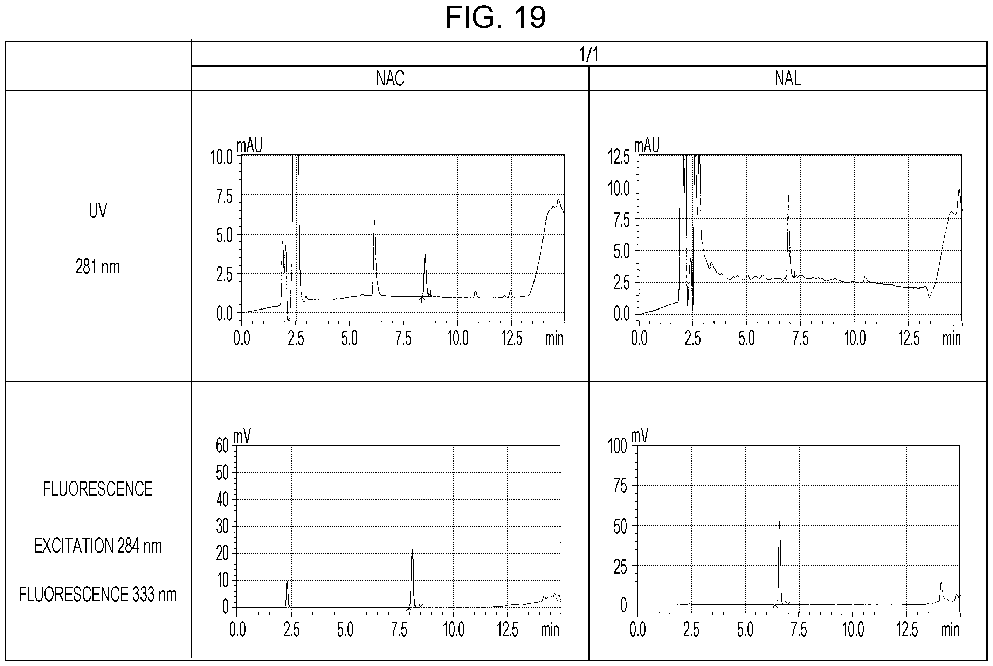

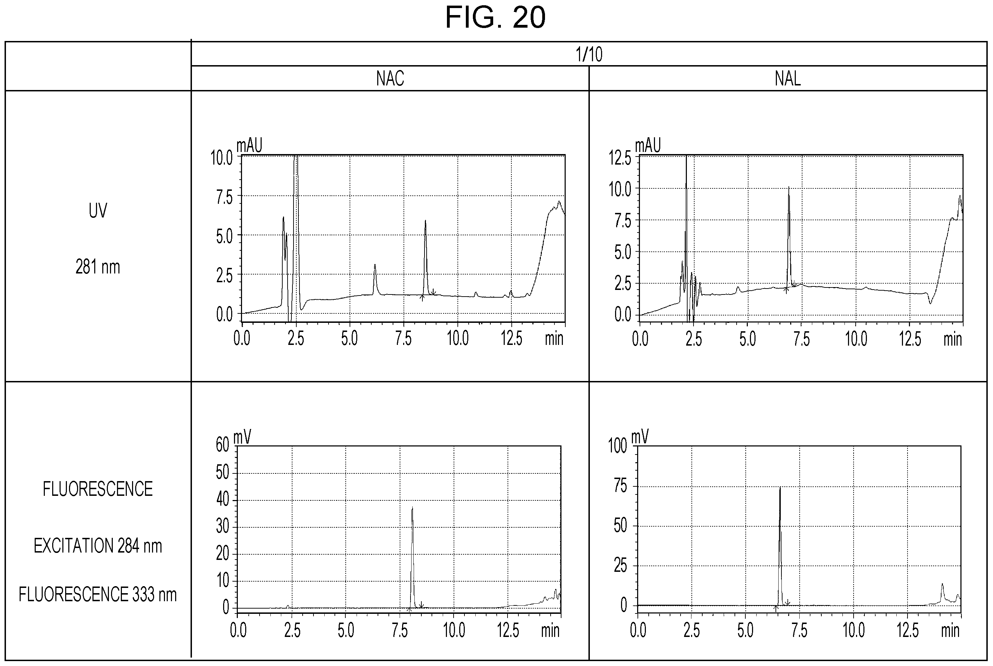

Fig. 19 and Fig. 20 include HPLC charts by UV detection and fluorescence detection for the reaction solution of protocatechuic acid or its diluted solution and a nucleophilic reagent (NAC or NAL).

In the HPLC measurement of the 20-component solution mixture of the aloe extract and its 1/10 diluted solution, in the case of UV detection at 281 nm, coelution for the peaks of the components and the nucleophilic reagents (NAC and NAL) occurred to some extent, but did not hinder quantification; however, depending on HPLC conditions, the quantification can be hindered. On the other hand, in the case of subjecting these to HPLC-fluorescence detection, most of the multiple components did not emit fluorescence, and hence the baseline was very low and stable. As a result, the peaks of the nucleophilic reagents (NAC and NAL) alone were detected, and accurate quantification was achieved without being affected by the multiple components.

β-sitosterol subjected to HPLC measurement exhibited a single peak at 281 nm, but did not coelute with the nucleophilic reagent, which has demonstrated that it is quantified even by the HPLC-UV method.

On the other hand, β-sitosterol subjected to fluorescence detection exhibited no peaks, and the nucleophilic reagent was quantified as a single peak.

Protocatechuic acid subjected to HPLC measurement basically behaved as with the β-sitosterol for both of the HPLC-UV method and the HPLC-fluorescence method; however, for the prepared undiluted solution, slight coelution with NAL occurred. The depletion of NAC was high, so that protocatechuic acid was found to partially react with NAC.

Prediction of skin allergenicity

From the HPLC charts in Fig. 9 to Fig. 20, the depletion percents (depletion) of the nucleophilic reagents were calculated, and the average scores were determined. From these values, skin allergenicity was predicted and the results are as follows.

(1) 20-component solution mixture of aloe extract

Table 4

| Detection method | 1/1 | | | | | 1/10 | | | | |
|---|---|---|---|---|---|---|---|---|---|---|
| | NAC | | NAL | | Mean Depletion (%) | NAC | | NAL | | Mean Depletion (%) |
| | Depletion (%) | SD | Depletion (%) | SD | | Depletion (%) | SD | Depletion (%) | SD | |
| UV method | 41.4 | 0.8 | 0.0 | 0.0 | 20.7 | 34.1 | 1.5 | 12.1 | 0.4 | 23.1 |
| Fluorescence method | 45.9 | 1.2 | 1.8 | 0.3 | 23.9 | 35.7 | 2.5 | 10.9 | 0.6 | 23.3 |

As is understood from the HPLC charts, for the 20-component solution mixture of the aloe extract and its 1/10 diluted solution, depletion of the nucleophilic reagents was calculated by both of the UV detection method and the fluorescence method. The solution mixture and the 1/10 diluted solution measured by the UV method were respectively found to have average scores of 20.7% and 23.1%, and hence both were determined as having skin allergenicity. On the other hand, they were measured by the fluorescence method and respectively found to have average scores of 23.9% and 23.3%, and hence both were similarly determined as having skin allergenicity.

Discussion

The 20-component solution mixture of the aloe extract and its 1/10 diluted solution measured by the UV method and the fluorescence method were found to have average scores of about 20%, and the average scores matched except for minor error. This has demonstrated that the fluorescence detection method enables measurement at the same accuracy as in the UV detection method. Protocatechuic acid in the 20 components of the aloe extract is a catechol derivative, and hence was inferred as a skin allergenic substance (pro-hapten). In fact, as described later, protocatechuic acid was determined as a skin allergenic substance; thus, the prediction that the 20-component solution mixture including the protocatechuic acid has skin allergenicity was correct.

(2) β-sitosterol and protocatechuic acid

Table 5

| Test substance | Detection method | 1/1 | | | | |
| | | NAC | | NAL | | Mean depletion (%) |
| | | Depletion (%) | SD | Depletion (%) | SD | |
|---|---|---|---|---|---|---|
| β-sitosterol | UV method | 0.0 | 0.0 | 0.0 | 0.0 | 0.0 |
| | Fluorescence method | 0.0 | 0.0 | 0.1 | 0.2 | 0.1 |
| Protocatechuic acid | UV method | 60.8 | 1.6 | 18.6 | 0.5 | 39.7 |
| | Fluorescence method | 58.1 | 1.0 | 38.3 | 0.2 | 48.2 |

| Test substance | Detection method | 1/10 | | | | |
| | | NAC | | NAL | | Mean Depletion (%) |
| | | Depletion (%) | SD | Depletion (%) | SD | |
|---|---|---|---|---|---|---|
| β-sitosterol | UV method | 3.9 | 6.7 | 0.0 | 0.0 | 1.9 |
| | Fluorescence method | 0.6 | 1.0 | 0.0 | 0.0 | 0.3 |
| Protocatechuic acid | UV method | 30.1 | 2.5 | 11.9 | 0.2 | 21.0 |
| | Fluorescence method | 27.3 | 1.1 | 11.5 | 0.4 | 19.4 |

As is understood from the HPLC charts, for the β-sitosterol and protocatechuic acid mono-component solutions and their 1/10 diluted solutions, depletion of the nucleophilic reagents was determined by both of the UV detection method and the fluorescence method.

Regarding the β-sitosterol solution, the mono-component solution and its 1/10 diluted solution measured by the UV method were respectively found to have average scores of 0.0% and 1.9%, and hence were both determined as not having skin allergenicity. On the other hand, they were measured by the fluorescence method and respectively found to have average scores of 0.1% and 0.3%, and hence were similarly both determined as not having skin allergenicity.

Regarding the protocatechuic acid solution, the mono-component solution and its 1/10 diluted solution measured by the UV method were respectively found to have average scores of 39.7% and 21.0%, and hence were both determined as having skin allergenicity. On the other hand, they were measured by the fluorescence method and respectively found to have average scores of 48.2% and 19.4%, and hence were similarly both determined as having skin allergenicity. Incidentally, regarding NAL in the prepared undiluted solution, coelution was slightly observed, and, because of the coelution, the calculated average score of 39.7% has low reliability (quantification accuracy).

Discussion

As described above, in the prepared undiluted solution of the protocatechuic acid mono-component solution, coelution with NAL occurred and hence the depletion of NAL and the average score have low reliability. Except for this, the average scores of the β-sitosterol and protocatechuic acid mono-component solutions and their 1/10 diluted solutions measured by the UV method and the fluorescence method were found to match except for minor errors of about 2% or less. This has demonstrated that the fluorescence detection method enables measurement at the same accuracy as in the UV detection method.

Incidentally, protocatechuic acid, which is a catechol derivative, was inferred as a skin allergenic substance (pro-hapten). In fact, protocatechuic acid was determined as a skin allergenic substance; thus, the major cause of the skin allergenicity of the 20-component solution mixture is inferred as protocatechuic acid.

Example 3

On the basis of reactivity between the nucleophilic reagent (NAC or NAL) and a test substance (average score or NAC depletion percent (depletion), skin allergenicity can be predicted. Thus, reference values (criteria) by which allergenicity and non-allergenicity are distinguished from each other were defined. Regarding a total of 82 substances (refer to the following Tables) composed of 53 known allergenic substances (18 strongly allergenic substances, 20 medium allergenic substances, and 15 weakly allergenic substances) and 29 known non-allergenic substances, the fluorescence method was performed and the depletion and average score of NAC and NAL were determined. The results are described below. In the following Tables, S represents as being an allergenic substance and NS represents as being a non-allergenic substance. The average scores of about 2/3 of the substances, that are 56 substances, were calculated by a 2-class classification method of an analysis software ADMEWORKS (FUJITSU KYUSHU SYSTEMS LIMITED), so that a reference value (criterion) for distinguishing allergenicity and non-allergenicity from each other was found to be 4.9%. Similarly, the depletion of NAC alone was calculated by the 2-class classification method, so that a criterion was found to be 5.6%.

List of test substances (82 substances)

Table 6

| No. | Test substance | EC3 (%) | CAS No. | Supplier | Solvent |
|---|---|---|---|---|---|
| Strongly allergenic substance | | | | | |
| 1 | Diphenylcyclopropenone | 0.0003 | 886-38-4 | FUJIFILM Wako Pure Chemical Corporation | Acetonitrile |
| 2 | Oxazolone | 0.003 | 15646-46-5 | FUJIFILM Wako Pure Chemical Corporation | Acetonitrile |
| 3 | Benzyl peroxide | 0.004 | 94-36-0 | Tokyo Chemical Industry Co., Ltd. | Acetonitrile |
| 4 | Kathon CG | 0.008 | 56965-84-9 | Sigma-Aldrich Corporation | Water |
| 5 | Bandrowski's base | 0.008 | 20048-27-5 | Alfa Aesar | 5% DMSO |
| 6 | 5-Chloro-methyl-4-isothiazolin-3-one | 0.009 | 26172-55-4 | Santa Cruz Biotechnology, Inc. | Water |
| 7 | p-Benzoquinone | 0.0099 | 106-51-4 | FUJIFILM Wako Pure Chemical Corporation | Acetonitrile |
| 8 | Tetrachlorosalicylanilide | 0.04 | 1154-59-2 | AccuStandard, Inc. | 5% DMSO |
| 9 | 2,4-Dinitrochlorobenzene | 0.05 | 97-00-7 | FUJIFILM Wako Pure Chemical Corporation | Acetonitrile |
| 10 | Glutaraldehyde | 0.1 | 111-30-8 | FUJIFILM Wako Pure Chemical Corporation | Water |
| 11 | Fluorescein isothiocyanate | 0.14 | 3326-32-7 | DOJINDO LABORATORIES | 5% DMSO |

(continued)

| No. | Test substance | EC3 (%) | CAS No. | Supplier | Solvent |
|---|---|---|---|---|---|
| Strongly allergenic substance | | | | | |
| 12 | Phthalic anhydride | 0.16 | 85-44-9 | FUJIFILM Wako Pure Chemical Corporation | Acetonitrile |
| 13 | Lauryl gallate | 0.3 | 1166-52-5 | FUJIFILM Wako Pure Chemical Corporation | 5% DMSO |
| 14 | Propyl gallate | 0.32 | 121-79-9 | FUJIFILM Wako Pure Chemical Corporation | Acetonitrile |
| 15 | CD3 | 0.6 | 25646-71-3 | FUJIFILM Corporation | Water |
| 16 | Trimellitic anhydride | 0.6 | 552-30-7 | FUJIFILM Wako Pure Chemical Corporation | Acetonitrile |
| 17 | Formaldehyde | 0.61 | 50-00-0 | FUJIFILM Wako Pure Chemical Corporation | Acetonitrile |
| 18 | Metol | 0.8 | 55-55-0 | FUJIFILM Wako Pure Chemical Corporation | Water |

Table 7

| No. | Test substance | EC3 (%) | CAS No. | Supplier | Solvent |
|---|---|---|---|---|---|
| Medium allergenic substance | | | | | |
| 19 | 2-Hydroxyethyl acrylate | 1.4 | 818-61-1 | FUJIFILM Wako Pure Chemical Corporation | Acetonitrile |
| 20 | Glyoxal | 1.4 | 107-22-2 | FUJIFILM Wako Pure Chemical Corporation | Water |
| 21 | Vinylpyridine | 1.6 | 1337-81-1 | FUJIFILM Wako Pure Chemical Corporation | Acetonitrile |
| 22 | 2-Mercaptobenzothiazole | 1.7 | 149-30-4 | FUJIFILM Wako Pure Chemical Corporation | 5% DMSO |
| 23 | Nonanoyl chloride | 1.8 | 764-85-2 | Tokyo Chemical Industry Co., Ltd. | Acetonitrile |
| 24 | 2-Methyl-2H-isothiazol-3-one | 1.9 | 2682-20-4 | Sigma-Aldrich Corporation | Acetonitrile |
| 25 | 1,2-Benzisothiazolin-3-one | 2.3 | 2634-33-5 | Tokyo Chemical Industry Co., Ltd. | 5% DMSO |
| 26 | Methyl 2-nonynoate | 2.5 | 111-80-8 | Tokyo Chemical Industry Co., Ltd. | Acetonitrile |
| 27 | Cinnamaldehyde | 3 | 14371-10-9 | FUJIFILM Wako Pure Chemical Corporation | Acetonitrile |
| 28 | Phenylacetaldehyde | 3 | 122-78-1 | Alfa Aesar | Acetonitrile |
| 29 | Benzylideneacetone | 3.7 | 122-57-6 | FUJIFILM Wako Pure Chemical Corporation | Acetonitrile |
| 30 | 2,4-Heptadienal | 4 | 881395 | FUJIFILM Wako Pure Chemical Corporation | Acetonitrile |
| 31 | Squaric acid | 4.3 | 2892-51-5 | FUJIFILM Wako Pure Chemical Corporation | Water |
| 32 | trans-2-Hexanal | 5.5 | 6728-26-3 | FUJIFILM Wako Pure Chemical Corporation | Acetonitrile |
| 33 | Resorcinol | 5.5 | 108-46-3 | FUJIFILM Wako Pure Chemical Corporation | Acetonitrile |
| 34 | Diethyl maleate | 5.8 | 141-05-9 | FUJIFILM Wako Pure Chemical Corporation | Acetonitrile |
| 35 | 2-Phenylpropionaldehyde | 6.3 | 93-53-8 | Sigma-Aldrich Corporation | Acetonitrile |
| 36 | Perillaldehyde | 8.1 | 2111-75-3 | FUJIFILM Wako Pure Chemical Corporation | Acetonitrile |

(continued)

| No. | Test substance | EC3 (%) | CAS No. | Supplier | Solvent |
|---|---|---|---|---|---|
| Medium allergenic substance | | | | | |
| 37 | Palmitoyl chloride | 8.8 | 112-67-4 | FUJIFILM Wako Pure Chemical Corporation | Acetone |
| 38 | 1-(4-Methoxyphenyl)-1-penten-3-one | 9.3 | 104-27-8 | AccuStandard, Inc. | Acetonitrile |

Table 8

| No. | Test substance | EC3 (%) | CAS No. | Supplier | Solvent |
|---|---|---|---|---|---|
| Weakly allergenic substance | | | | | |
| 39 | α-Hexylcinnamaldehyde | 11 | 101-86-0 | FUJIFILM Wako Pure Chemical Corporation | Acetonitrile |
| 40 | α-Amylcinnamaldehyde | 11 | 122-40-7 | FUJIFILM Wako Pure Chemical Corporation | Acetonitrile |
| 41 | 2,3-Butanedione | 11 | 431-03-8 | FUJIFILM Wako Pure Chemical Corporation | Acetonitrile |
| 42 | Farnesal | 12 | 19317-11-4 | Frinton Laboratories | Acetonitrile |
| 43 | Oxalic acid | 15 | 144-62-7 | FUJIFILM Wako Pure Chemical Corporation | Acetonitrile |
| 44 | Benzyl benzoate | 17 | 120-51-4 | FUJIFILM Wako Pure Chemical Corporation | Acetonitrile |
| 45 | 4-Allylanisole | 18 | 140-67-0 | Tokyo Chemical Industry Co., Ltd. | Acetonitrile |
| 46 | Lilial | 19 | 80-54-6 | FUJIFILM Wako Pure Chemical Corporation | Acetonitrile |
| 47 | Cyclamen aldehyde | 22 | 103-95-7 | Sigma-Aldrich Corporation | Acetonitrile |
| 48 | Imidazolidinyl urea | 24 | 39236-46-9 | Sigma-Aldrich Corporation | Water |
| 49 | 5- Methyl-2,3 -hexanedione | 26 | 13706-86-0 | Tokyo Chemical Industry Co., Ltd. | Acetonitrile |
| 50 | 2,2,6,6-Tetramethyl-3,5-heptanedione | 27 | 1118-71-4 | Tokyo Chemical Industry Co., Ltd. | Acetonitrile |
| 51 | Ethylene glycol dimethacrylate | 28 | 97-90-5 | FUJIFILM Wako Pure Chemical Corporation | Acetonitrile |
| 52 | Ethyl acrylate | 28 | 140-88-5 | FUJIFILM Wako Pure Chemical Corporation | Acetonitrile |
| 53 | Hydroxycitronellal | 33 | 107-75-5 | FUJIFILM Wako Pure Chemical Corporation | Acetonitrile |

| No. | Test substance | EC3 (%) | CAS No. | Supplier | Solvent |
|---|---|---|---|---|---|
| Non-allergenic substance | | | | | |
| 54 | Glycerol | - | 56-81-5 | FUJIFILM Wako Pure Chemical Corporation | Acetonitrile |
| 55 | Hexane | - | 110-54-3 | FUJIFILM Wako Pure Chemical Corporation | Acetonitrile |
| 56 | Diethyl phthalate | - | 84-66-2 | FUJIFILM Wako Pure Chemical Corporation | Acetonitrile |
| 57 | Octanoic acid | - | 124-07-2 | FUJIFILM Wako Pure Chemical Corporation | Acetonitrile |
| 58 | 2-Hydroxypropyl methacrylate | - | 923-26-2 | FUJIFILM Wako Pure Chemical Corporation | Acetonitrile |
| 59 | 1-Butanol | - | 71-36-3 | FUJIFILM Wako Pure Chemical Corporation | Acetonitrile |
| 60 | 4-Hydroxybenzoic acid | - | 99-96-7 | FUJIFILM Wako Pure Chemical Corporation | Acetonitrile |
| 61 | 6-Methylcoumarin | - | 92-48-8 | Sigma-Aldrich Corporation | Acetonitrile |

(continued)

| No. | Test substance | EC3 (%) | CAS No. | Supplier | Solvent |
|---|---|---|---|---|---|
| Non-allergenic substance | | | | | |
| 62 | Methyl salicylate | - | 119-36-8 | FUJIFILM Wako Pure Chemical Corporation | Acetonitrile |
| 63 | Chlorobenzene | - | 108-90-7 | FUJIFILM Wako Pure Chemical Corporation | Acetonitrile |
| 64 | Lactic acid | - | 50-21-5 | Sigma-Aldrich Corporation | Acetonitrile |
| 65 | 1-Bromobutane | - | 109-65-9 | FUJIFILM Wako Pure Chemical Corporation | Acetonitrile |
| 66 | 2-Acetylcyclohexanone | - | 874-23-7 | FUJIFILM Wako Pure Chemical Corporation | Acetonitrile |
| 67 | 4'-Methoxyacetophenone | - | 100-06-1 | FUJIFILM Wako Pure Chemical Corporation | Acetonitrile |
| 68 | Ethylbenzyl acetate | - | 94-02-0 | FUJIFILM Wako Pure Chemical Corporation | Acetonitrile |
| 69 | Ethyl vanillin | - | 121-32-4 | Tokyo Chemical Industry Co., Ltd. | Acetonitrile |
| 70 | Isopropanol | - | 67-63-0 | FUJIFILM Wako Pure Chemical Corporation | Acetonitrile |
| 71 | Propylene glycol | - | 57-55-6 | FUJIFILM Wako Pure Chemical Corporation | Acetonitrile |
| 72 | Sulfanilamide | - | 63-74-1 | FUJIFILM Wako Pure Chemical Corporation | Acetonitrile |
| 73 | Isopropyl myristate | - | 110-27-0 | FUJIFILM Wako Pure Chemical Corporation | Acetonitrile |

Table 9

| No. | Test substance | EC3 (%) | CAS No. | Supplier | Solvent |
|---|---|---|---|---|---|
| 74 | Benzaldehyde | - | 100-52-7 | Sigma-Aldrich Corporation | Acetonitrile |
| 75 | Methylparaben | - | 99-76-3 | FUJIFILM Wako Pure Chemical Corporation | Acetonitrile |
| 76 | Nonanoic acid | 21 (False+) | 112-05-0 | Tokyo Chemical Industry Co., Ltd. | Acetonitrile |
| 77 | Propylparaben | - | 94-13-3 | FUJIFILM Wako Pure Chemical Corporation | Acetonitrile |
| 78 | Salicylic acid | - | 69-72-7 | FUJIFILM Wako Pure Chemical Corporation | Acetonitrile |
| 79 | Sulfanilic acid | - | 121-57-3 | FUJIFILM Wako Pure Chemical Corporation | Water |
| 80 | Vanillin | - | 121-33-5 | FUJIFILM Wako Pure Chemical Corporation | Acetonitrile |
| 81 | Coumarin | - | 91-64-5 | FUJIFILM Wako Pure Chemical Corporation | Acetonitrile |
| 82 | Benzylidene dichloride | - | 75-35-4 | AccuStandard, Inc. | Acetonitrile |

Test results

(1) Strongly allergenic substance

Table 10

| No. | Test substance | Fluorescence method | | | | Reference (UV method) | | | |
|-----|----------------|-----|-----|-----|-----|-----|-----|-----|-----|
| | | NAC depletion (%) | NAL depletion (%) | Average score | Prediction result | NAC depletion (%) | depletion (%) | Average score | Prediction result |
| Strongly allergenic substance | | | | | | | | | |
| 1 | Diphenylcyclopropenone | 29.7 | 2.4 | 16.0 | S | 28.7 | 6.3 | 17.5 | S |
| 2 | Oxazolone | 92.0 | 65.5 | 78.8 | S | 85.3 | 80.1 | 82.7 | S |
| 3 | Benzyl peroxide | 100.0 | 63.7 | 81.9 | S | 100.0 | 50.6 | 75.3 | S |
| 4 | Kathon CG | 100.0 | 2.0 | 51.0 | S | 100.0 | -0.4 | 49.8 | S |
| 5 | Bandrowski's base | 100.0 | 1.7 | 50.8 | S | 100.0 | 5.7 | 52.9 | S |
| 6 | 5 -Chloro-methyl-4-isothiazolin-3-one | 100.0 | 9.3 | 54.7 | S | 100.0 | 17.7 | 58.8 | S |
| 7 | p-Benzoquinone | 100.0 | 71.8 | 85.9 | S | 96.5 | 83.5 | 90.0 | S |
| 8 | Tetrachlorosalicylanilide | 44.5 | 1.2 | 22.9 | S | 43.4 | 2.1 | 22.7 | S |
| 9 | 2,4-Dinitrochlorobenzene | 91.6 | 0.0 | 45.8 | S | 89.3 | 6.1 | 47.7 | S |
| 10 | Glutaraldehyde | 0.0 | 47.4 | 23.7 | S | 0.8 | 53.1 | 26.9 | S |
| 11 | Fluorescein isothiocyanate | 81.3 | 97.6 | 89.4 | S | 73.6 | 98.1 | 85.8 | S |
| 12 | Phthalic anhydride | 0.3 | 92.6 | 46.4 | S | 0.3 | 96.9 | 48.6 | S |
| 13 | Lauryl gallate | 99.5 | 15.9 | 57.7 | S | 100.0 | 19.0 | 59.5 | S |
| 14 | Propyl gallate | 100.0 | 65.3 | 82.6 | S | 97.1 | 56.4 | 76.7 | S |
| 15 | CD3 | 100.0 | 8.1 | 54.0 | S | 76.6 | 16.5 | 46.6 | S |
| 16 | Trimellitic anhydride | 0.8 | 94.2 | 47.5 | S | 1.9 | 97.0 | 49.4 | S |
| 17 | Formaldehyde | 19.6 | 2.5 | 11.0 | S | 25.7 | 1.6 | 13.6 | S |
| 18 | Metol | 100.0 | 15.9 | 57.9 | S | 100.0 | 22.8 | 61.4 | S |

(2) Medium allergenic substance

Table 11

| No. | Test substance | Fluorescence method | | | | Reference (UV method) | | | |
|---|---|---|---|---|---|---|---|---|---|
| | | NAC depletion (%) | NAL depletion (%) | Average score | Prediction result | NAC depletion (%) | NAL depletion (%) | Average score | Prediction result |
| Medium allergenic substance | | | | | | | | | |
| 19 | 2-Hydroxyethyl acrylate | 100.0 | 19.7 | 59.9 | S | 100.0 | 16.3 | 58.1 | S |
| 20 | Glyoxal | 24.9 | 0.4 | 12.7 | S | 12.8 | 0.8 | 6.8 | S |
| 21 | Vinylpyridine | 19.8 | 3.6 | 11.7 | S | 18.1 | 7.7 | 12.9 | S |
| 22 | 2-Mercaptobenzothiazole | 65.5 | 0.0 | 32.7 | S | 56.6 | 0.3 | 28.4 | S |
| 23 | Nonanoyl chloride | 8.9 | 54.5 | 31.7 | S | 7.4 | 39.4 | 23.4 | S |
| 24 | 2-Methyl-2H-isothiazol-3-one | 98.5 | 4.6 | 51.6 | S | 94.0 | 7.0 | 50.5 | S |
| 25 | 1,2-Benzisothiazolin-3-one | 98.8 | 0.2 | 49.5 | S | 100.0 | 0.4 | 50.2 | S |
| 26 | Methyl-2-nonynoate | 12.4 | 3.2 | 7.8 | S | 15.0 | 1.4 | 8.2 | S |
| 27 | Cinnamaldehyde | 54.1 | 12.8 | 33.4 | S | 37.0 | 13.1 | 25.0 | S |
| 28 | Phenylacetaldehyde | 9.5 | 96.2 | 52.9 | S | 22.4 | 98.3 | 60.3 | S |
| 29 | Benzylideneacetone | 67.3 | 10.0 | 38.6 | S | 44.9 | 7.2 | 26.1 | S |
| 30 | 2,4-Heptadienal | 46.2 | 11.8 | 29.0 | S | 35.8 | 50.1 | 42.9 | S |
| 31 | Squaric acid | 1.0 | 0.1 | 0.5 | NS | 0.8 | 0.5 | 0.6 | NS |
| 32 | trans-2-Hexanal | 97.9 | 11.4 | 54.6 | S | 80.4 | 12.3 | 46.3 | S |
| 33 | Resorcinol | 0.8 | 1.6 | 1.2 | NS | 4.0 | 2.2 | 3.1 | NS |
| 34 | Diethyl maleate | 31.2 | 4.7 | 18.0 | S | 31.8 | 7.7 | 19.8 | S |
| 35 | 2-Phenylpropionaldehyde | 8.5 | 7.3 | 7.9 | S | 8.0 | 4.6 | 6.3 | S |
| 36 | Perillaldehyde | 42.4 | 7.1 | 24.7 | S | 29.1 | 18.5 | 23.8 | S |
| 37 | Palmitoyl chloride | 11.5 | 98.3 | 54.9 | S | 5.8 | 95.9 | 50.8 | S |
| 38 | 1-(4-Methoxyphenyl)-1-penten-3-one | 1.2 | 0.4 | 0.8 | NS | 1.6 | 4.2 | 2.9 | NS |

(3) Weakly allergenic substance

| No. | Test substance | Fluorescence method | | | | Reference (UV method) | | | |
|---|---|---|---|---|---|---|---|---|---|
| | | NAC depletion (%) | NAL depletion (%) | Average score | Prediction result | NAC depletion (%) | NAL depletion (%) | Average score | Prediction result |
| Weakly allergenic substance | | | | | | | | | |
| 39 | α-Hexylcinnamaldehyde | 0.0 | 0.0 | 0.0 | NS | 0.0 | 1.1 | 0.6 | NS |
| 40 | α-Amylcinnamaldehyde | 0.3 | 0.4 | 0.3 | NS | 0.0 | 1.6 | 0.8 | NS |

Table 12

| 41 | 2,3-Butanedione | 45.2 | 17.7 | 31.4 | S | 41.2 | 25.5 | 33.4 | S |
| 42 | Farnesal | 22.4 | 4.9 | 13.6 | S | 17.2 | 8.8 | 13.0 | S |
| 43 | Oxalic acid | 0.0 | 0.3 | 0.1 | NS | 0.0 | 3.6 | 1.8 | NS |
| 44 | Benzyl benzoate | 0.0 | 0.3 | 0.1 | NS | 0.0 | 2.8 | 1.4 | NS |
| 45 | 4-Allylanisole | 15.0 | 0.7 | 7.9 | S | 27.0 | 3.9 | 15.4 | S |
| 46 | Lilial | 18.0 | 0.2 | 9.1 | S | 7.0 | 3.9 | 5.4 | S |
| 47 | Cyclamen aldehyde | 12.2 | 0.0 | 6.1 | S | 3.1 | 1.7 | 2.4 | NS |
| 48 | Imidazolidinyl urea | 23.2 | 6.3 | 14.8 | S | 35.4 | 2.0 | 18.7 | S |
| 49 | 5- Methyl-2,3 -hexanedione | 14.8 | 26.1 | 20.4 | S | 6.4 | 34.8 | 20.6 | S |
| 50 | 2,2,6,6-Tetramethyl-3,5-heptanedione | 0.0 | 0.0 | 0.0 | NS | 0.5 | 1.6 | 1.1 | NS |
| 51 | Ethylene glycol dimethacrylate | 7.3 | 1.5 | 4.4 | NS | 5.9 | 2.6 | 4.3 | NS |
| 52 | Ethyl acrylate | 88.7 | 10.6 | 49.6 | S | 87.8 | 13.7 | 50.8 | S |
| 53 | Hydroxycitronellal | 16.0 | 0.0 | 8.0 | S | 17.5 | 1.5 | 9.5 | S |

(4) Non-allergenic substance

Table 13

| No | Test substance | Fluorescence method | | | | Reference (UV method) | | | |
|---|---|---|---|---|---|---|---|---|---|
| | | NAC depletio n (%) | NAL depletio n (%) | Averag e score | Predictio n result | NAC depletio n (%) | NAL depletio n (%) | Averag e score | Predictio n result |
| Non-allergenic substance | | | | | | | | | |
| 54 | Glycerol | 0.2 | 0.0 | 0.1 | NS | 0.3 | 0.7 | 0.5 | NS |
| 55 | Hexane | 0.0 | 0.0 | 0.0 | NS | 0.2 | 0.9 | 0.5 | NS |
| 56 | Diethyl phthalate | 0.0 | 0.2 | 0.1 | NS | 0.0 | 1.7 | 0.9 | NS |
| 57 | Octanoic acid | 0.0 | 0.0 | 0.0 | NS | 0.0 | 1.2 | 0.6 | NS |
| 58 | 2-Hydroxypropyl methacrylate | 2.0 | 0.2 | 1.1 | NS | 2.4 | 2.2 | 2.3 | NS |
| 59 | 1-Butanol | 2.1 | 0.3 | 1.2 | NS | 0.1 | 0.8 | 0.5 | NS |
| 60 | 4-Hydroxybenzoic acid | 2.9 | 0.1 | 1.5 | NS | 0.0 | -1.4 | -0.7 | NS |
| 61 | 6-Methylcoumarin | 1.3 | 0.1 | 0.7 | NS | 0.0 | 1.0 | 0.5 | NS |
| 62 | Methyl salicylate | 0.3 | 0.0 | 0.1 | NS | 0.0 | 0.4 | 0.2 | NS |
| 63 | Chlorobenzene | 1.3 | 0.1 | 0.7 | NS | 0.0 | 0.7 | 0.4 | NS |
| 64 | Lactic acid | 1.4 | 0.0 | 0.7 | NS | 0.0 | 2.8 | 1.4 | NS |
| 65 | 1-Bromobutane | 0.0 | 0.0 | 0.0 | NS | 0.1 | 2.4 | 1.2 | NS |
| 66 | 2-Acetylcyclohexanone | 3.5 | 0.0 | 1.7 | NS | 1.3 | 0.3 | 0.8 | NS |
| 67 | 4'-Methoxyacetophenon e | 1.2 | 0.0 | 0.6 | NS | 3.3 | 3.3 | 3.3 | NS |
| 68 | Ethylbenzyl acetate | 3.7 | 0.0 | 1.9 | NS | 4.4 | 5.3 | 4.9 | NS |
| 69 | Ethyl vanillin | 1.8 | 4.9 | 3.4 | NS | 1.4 | 2.1 | 1.7 | NS |
| 70 | Isopropanol | 1.1 | 4.6 | 2.8 | NS | 0.1 | 2.8 | 1.5 | NS |
| 71 | Propylene glycol | 0.7 | 5.0 | 2.8 | NS | 0.1 | 2.2 | 1.1 | NS |
| 72 | Sulfanilamide | 0.5 | 3.6 | 2.1 | NS | 0.0 | 1.4 | 0.7 | NS |
| 73 | Isopropyl myristate | 0.4 | 5.8 | 3.1 | NS | 0.0 | 1.8 | 0.9 | NS |
| 74 | Benzaldehyde | 29.6 | 7.9 | 18.8 | S | 25.3 | 2.9 | 14.1 | S |
| 75 | Methylparaben | 0.7 | 4.7 | 2.7 | NS | 0.1 | 1.3 | 0.7 | NS |
| 76 | Nonanoic acid | 0.0 | 0.0 | 0.0 | NS | 0.0 | 1.3 | 0.6 | NS |
| 77 | Propylparaben | 0.5 | 4.4 | 2.5 | NS | 0.1 | 1.9 | 1.0 | NS |
| 78 | Salicylic acid | 0.1 | 4.3 | 2.2 | NS | 0.0 | 0.4 | 0.2 | NS |
| 79 | Sulfanilic acid | 0.0 | 0.1 | 0.1 | NS | 0.5 | 0.4 | 0.5 | NS |
| 80 | Vanillin | 2.0 | 0.0 | 1.0 | NS | 1.5 | 1.9 | 1.7 | NS |
| 81 | Coumarin | 1.2 | 0.4 | 0.8 | NS | 0.5 | 3.9 | 2.2 | NS |

| No | Test substance | Fluorescence method | | | | Reference (UV method) | | | |
|---|---|---|---|---|---|---|---|---|---|
| | | NAC depletion (%) | NAL depletion (%) | Average score | Prediction result | NAC depletion (%) | NAL depletion (%) | Average score | Prediction result |
| Non-allergenic substance | | | | | | | | | |
| 82 | Benzylidene dichloride | 2.1 | 1.4 | 1.7 | NS | 0.9 | -0.3 | 0.3 | NS |

(5) Prediction accuracy

Table 14

| | | Prediction-based classification | | | | | |
| --- | --- | --- | --- | --- | --- | --- | --- |
| | | Fluorescence method | | | Reference (UV method) | | |
| | | Allergenic substance | Non-allergenic substance | Total | Allergenic substance | Non-allergenic substance | Total |
| LLNA-test-based classification | Allergenic substance | 44 | 9 | 53 | 43 | 10 | 53 |
| | Non-allergenic substance | 1 | 28 | 29 | 1 | 28 | 29 |
| | Total | 45 | 37 | 82 | 44 | 38 | 82 |
| | Sensitivity: | | | 83% | Sensitivity: | | 81% |
| | Specificity: | | | 97% | Specificity: | | 97% |
| | Prediction accuracy: | | | 88% | Prediction accuracy: | | 87% |

As is understood from the results, the test results obtained by the fluorescence method and the prediction accuracy for skin allergenicity based on the criterion of 4.9% were substantially perfect reproduction of the results obtained by the UV method. These results have demonstrated that the fluorescence method is usable as with the UV method.

**Claims**

**1.** A method for determining skin allergenicity, the method comprising:

causing a reaction between an organic compound that has at least one thiol group or amino group and emits fluorescence, and a mixture containing two or more test substances,
wherein the organic compound is N-(arylalkylcarbonyl)cysteine or $\alpha$-N-(arylalkylcarbonyl)lysine, and the organic compound is an organic compound that emits fluorescence at 200 to 400 nm, and has a molar absorption coefficient at a maximal absorption wavelength of 10 L/mol·cm or more and 500000 L/mol·cm or less;
determining an amount of the organic compound after the reaction or an amount of a product of the reaction by an optical measurement using a fluorescence detector,
wherein, in the optical measurement using the fluorescence detector, an excitation wavelength is 200 to 350 nm, and a fluorescence wavelength is 200 to 400 nm;
subj ecting, to chromatography treatment, a reaction product of the step of causing the reaction between the organic compound and the mixture,
calculating, by a formula below, a depletion percent of the organic compound from an average value of peak areas of the organic compound determined by the optical measurement using the fluorescence detector, Depletion percent (% depletion) of organic compound that has at least one thiol group and emits fluorescence = [1 - (Average value of peak areas of post-reaction unreacted organic compound that has at least one thiol group and emits fluorescence/Average value of peak areas of blank-test organic compound that has at least one thiol group and emits fluorescence)] × 100;

or Depletion percent (% depletion) of organic compound that has at least one aminol group and emits fluorescence = [1 - (Average value of peak areas of post-reaction unreacted organic compound that has at least one thiol group and emits fluorescence/Average value of peak areas of blank-test organic compound that has at least one amino group and emits fluorescence)] $\times$ 100.

2. The method for determining skin allergenicity according to claim 1, wherein the organic compound is N-(2-phenylacetyl)cysteine or N-[2-(naphthalen-1-yl)acetyl]cysteine.

3. The method for determining skin allergenicity according to claim 1, wherein the organic compound is $\alpha$-N-(2-phenylacetyl)lysine or $\alpha$-N-[2-(naphthalen-1-yl)acetyl]lysine.

4. The method for determining skin allergenicity according to any one of claims 1 to 3, wherein the mixture is at least one selected from the group consisting of perfume, essential oil, polymers, pharmaceuticals, agricultural chemicals, foods, chemical products, and plant extracts composed of natural-product-derived components.

5. The method for determining skin allergenicity according to any one of claims 1 to 4, the method comprising calculating an average value of the depletion percent of the organic compound that has at least one thiol group and emits fluorescence and the depletion percent of the organic compound that has at least one amino group and emits fluorescence, or calculating the depletion percent of the organic compound that has at least one thiol group and emits fluorescence, and determining whether the test substances are allergenic substances or non-allergenic substances:

(1) in a case of performing evaluation based on the average value of the depletion percent of the organic compound that has at least one thiol group and emits fluorescence and the depletion percent of the organic compound that has at least one amino group and emits fluorescence,

Average score < 4.9%: the test substances are determined as non-allergenic substances,
Average score $\geq$ 4.9%: the test substances are determined as allergenic substances, or

(2) in a case of performing evaluation based on only the depletion percent of the organic compound that has at least one thiol group and emits fluorescence,

Depletion percent of organic compound that has at least one thiol group and emits fluorescence < 5.6%: the test substances are determined as non-allergenic substances,
Depletion percent of organic compound that has at least one thiol group and emits fluorescence $\geq$ 5.6%: the test substances are determined as allergenic substances.

**Patentansprüche**

1. Verfahren zur Bestimmung von Hautallergenität, wobei das Verfahren umfasst:

Verursachen einer Reaktion zwischen einer organischen Verbindung, die zumindest eine Thiolgruppe oder Aminogruppe aufweist und Fluoreszenz emittiert, und einer Mischung, die zwei oder mehr Testsubstanzen enthält,
worin die organische Verbindung N-(Arylalkylcarbonyl)cystein oder $\alpha$-N-(Arylalkylcarbonyl)lysin ist und die organische Verbindung eine organische Verbindung ist, die Fluoreszenz bei 200 bis 400 nm emittiert und einen molaren Absorptionskoeffizienten bei der maximalen Absorptionswellenlänge von 10 L/mol·cm oder größer und 500.000 L/mol·cm oder kleiner aufweist;
Bestimmen einer Menge der organischen Verbindung nach der Reaktion oder einer Menge eines Reaktionsprodukts durch eine optische Messung mit einem Fluoreszenzdetektor,
worin bei der optischen Messung mit dem Fluoreszenzdetektor eine Anregungswellenlänge 200 bis 350 nm beträgt und eine Fluoreszenzwellenlänge von 200 bis 400 nm beträgt;
Unterziehen eines Reaktionsprodukts aus dem Schritt zum Verursachen der Reaktion zwischen der organischen Verbindung und der Mischung unter eine Chromatographiebehandlung,
Berechnen eines Abreicherungs-Prozentwerts der organischen Verbindung aus einem Mittelwert der durch die optische Messung mit dem Fluoreszenzdetektor bestimmten Peakflächen der organischen Verbindung durch die nachstehende Formel, Abreicherungs-Prozentwert (%-Abreicherung) der organischen Verbindung, die zumindest eine Thiolgruppe aufweist und Fluoreszenz emittiert = [1 - (Mittelwert von Peakflächen von nicht-reagierter organischer Verbindung, die zumindest eine Thiolgruppe aufweist und Fluoreszenz emittiert, nach der Reaktion/Mittelwert von Peakflächen der organischen Verbindung, die zumindest eine Thiolgruppe aufweist und Fluoreszenz emittiert, als Blindprobe)] $\times$ 100;

oder

Abreicherungs-Prozentwert (%-Abreicherung) der organischen Verbindung, die zumindest eine Aminogruppe aufweist und Fluoreszenz emittiert = [1 - (Mittelwert von Peakflächen von nicht-reagierter organischer Verbindung, die zumindest eine Aminogruppe aufweist und Fluoreszenz emittiert, nach der Reaktion/Mittelwert von Peakflächen der organischen Verbindung, die zumindest eine Aminogruppe aufweist und Fluoreszenz emittiert, als Blindprobe)] $\times$ 100.

2. Verfahren zur Bestimmung von Hautallergenität gemäß Anspruch 1, worin die organische Verbindung N-(2-Phenylacetyl)cystein oder N-[2-(Naphthalin-1-yl)acetyl]cystein ist.

3. Verfahren zur Bestimmung von Hautallergenität gemäß Anspruch 1, worin die organische Verbindung $\alpha$-N-(2-Phenylacetyl)lysin oder a-N-[2-(Naphthalin-1-yl)acetyl]lysin ist.

4. Verfahren zur Bestimmung von Hautallergenität gemäß irgendeinem der Ansprüche 1 bis 3, worin die Mischung zumindest eine ist, die ausgewählt ist aus der Gruppe bestehend aus Parfüm, essentiellen Ölen, Polymeren, Parmazeutika, Agro-Chemikalien, Nahrungsmitteln, chemischen Produkten und Pflanzenextrakten, die aus Komponenten aus natürlichem Ursprung zusammengesetzt sind.

5. Verfahren zur Bestimmung von Hautallergenität gemäß irgendeinem der Ansprüche 1 bis 4, wobei das Verfahren die Berechnung eines Mittelwerts des Abreicherungs-Prozentwerts der organischen Verbindung, die zumindest eine Thiolgruppe aufweist und Fluoreszenz emittiert, und des Abreicherungs-Prozentwerts der organischen Verbindung, die zumindest eine Aminogruppe aufweist und Fluoreszenz emittiert, umfasst, oder das das Berechnen des Abreicherungs-Prozentwerts der organischen Verbindung, die zumindest eines Thiolgruppe aufweist und Fluoreszenz emittiert, und die Bestimmung, ob die Testsubstanzen allergene Substanzen oder nicht-allergene Substanzen sind, umfasst; wobei

(1) wenn die Untersuchung auf Grundlage des Mittelwerts des Abreicherungs-Prozentwerts der organischen Verbindung, die zumindest eine Thiolgruppe aufweist und Fluoreszenz emittiert, und des Abreicherungs-Prozentwerts der organischen Verbindung, die zumindest eine Aminogruppe aufweist und Fluoreszenz emittiert, durchgeführt wird,

Mittelwert < 4,9 %: die Testsubstanzen werden als nicht-allergene Substanzen bestimmt,
Mittelwert $\geq$ 4,9 %: die Testsubstanzen werden als allergene Substanzen bestimmt, oder

(2) wenn die Bewertung nur auf Grundlage des Abreicherungs-Prozentwerts der organischen Verbindung, die zumindest eine Thiolgruppe aufweist und Fluoreszenz emittiert, durchgeführt wird,

Abreicherungs-Prozent der organischen Verbindung, die zumindest eine Thiolgruppe aufweist und Fluoreszenz emittiert < 5,6 %: die Testsubstanzen werden als nicht-allergene Substanzen bestimmt,
Abreicherungs-Prozent der organischen Verbindung, die zumindest eine Thiolgruppe aufweist und Fluoreszenz emittiert $\geq$ 5,6 %: die Testsubstanzen werden als allergene Substanzen bestimmt.

**Revendications**

1. Procédé pour déterminer la sensibilisation de la peau, le procédé comprenant les étapes suivantes :

déclencher une réaction entre un composé organique qui présente au moins un groupe thiol ou un groupe amino et qui émet une fluorescence, et un mélange contenant deux substances de test ou plus,
dans lequel le composé organique est la N-(arylalkylcarbonyl)cystéine ou l'α-N-(arylalkylcarbonyl)lysine, et le composé organique est un composé organique qui émet une fluorescence comprise entre 200 et 400 nm, et qui présente un coefficient d'absorption molaire à une longueur d'onde d'absorption maximale de 10 L/mol-cm ou plus et de 500000 L/mol-cm ou moins ;
déterminer une quantité du composé organique après la réaction ou une quantité d'un produit de la réaction par une mesure optique en utilisant un détecteur de fluorescence,
dans lequel, dans la mesure optique réalisée en utilisant le détecteur de fluorescence, une longueur d'onde d'excitation est comprise entre 200 et 350 nm, et une longueur d'onde de fluorescence est comprise entre 200 et 400 nm ;
soumettre à un traitement de chromatographie un produit de réaction de l'étape de déclenchement de la réaction entre le composé organique et le mélange,
calculer, par une formule ci-dessous, un pourcentage d'épuisement du composé organique à partir d'une valeur moyenne de surfaces de pic du composé organique déterminée par la mesure optique utilisant le détecteur de fluorescence, pourcentage d'épuisement (% d'épuisement) du composé organique qui présente au moins un groupe thiol et qui émet une fluorescence = [1 - (Valeur moyenne des surfaces de pic du composé organique n'ayant pas réagi après réaction qui présente au moins un groupe thiol et qui émet une fluorescence/Valeur moyenne des surfaces de pic du composé organique de test à blanc qui présente au moins un groupe thiol et qui émet une fluorescence)] × 100 ;

ou pourcentage d'épuisement (% d'épuisement) du composé organique qui présente au moins un groupe amino et qui émet une fluorescence = [1 - (valeur moyenne des surfaces de pic du composé organique n'ayant pas réagi après réaction qui présente au moins un groupe amino et qui émet une fluorescence/valeur moyenne des surfaces de pic du composé organique de test à blanc qui présente au moins un groupe amino et qui émet une fluorescence)] × 100.

2. Procédé pour déterminer la sensibilisation de la peau selon la revendication 1, dans lequel le composé organique est la N-(2-phénylacétyl)cystéine ou la N-[2-(naphtalène-1-yl)acétyl]cystéine.

3. Procédé pour déterminer la sensibilisation de la peau selon la revendication 1, dans lequel le composé organique est l'α-N-(2-phénylacétyl)lysine ou l'α-N-[2-(naphthalen-1-yl)acétyl]lysine.

4. Procédé pour déterminer la sensibilisation de la peau selon l'une quelconque des revendications 1 à 3, dans lequel le mélange est au moins un produit sélectionné dans le groupe comprenant des parfums, des huiles essentielles, des polymères, des produits pharmaceutiques, des produits chimiques agricoles, des aliments, des produits chimiques et des extraits de plantes composés de composants dérivés de produits naturels.

5. Procédé pour déterminer la sensibilisation de la peau selon l'une quelconque des revendications 1 à 4, le procédé comprenant le calcul d'une valeur moyenne du pourcentage d'épuisement du composé organique qui présente au moins un groupe thiol et qui émet une fluorescence et du pourcentage d'épuisement du composé organique qui présente au moins un groupe amino et qui émet une fluorescence, ou le calcul du pourcentage d'épuisement du composé organique qui présente au moins un groupe thiol et qui émet une fluorescence, et la détermination si les substances de test sont des substances allergènes ou des substances non allergènes :

(1) dans le cas de la mise en oeuvre d'une évaluation basée sur la valeur moyenne du pourcentage d'épuisement du composé organique qui présente au moins un groupe thiol et qui émet une fluorescence et du pourcentage d'épuisement du composé organique qui présente au moins un groupe amino et qui émett une fluorescence,

résultat moyen < 4,9 % : les substances de test sont déterminées comme étant substances non allergènes,
résultat moyen >_ 4,9 % : les substances de test sont déterminées comme étant des substances allergènes, ou

(2) dans le cas de la mise en oeuvre une évaluation basée uniquement sur le pourcentage d'épuisement du composé organique qui présente au moins un groupe thiol et qui émet une fluorescence,

pourcentage d'épuisement du composé organique qui présente au moins un groupe thiol et qui émet une fluorescence < 5,6 % : les substances de test sont déterminées comme étant des substances non allergènes, pourcentage d'épuisement en composé organique qui présente au moins un groupe thiol et qui émet une fluorescence >_ 5,6 % : les substances de test sont déterminées comme étant des substances allergènes.

FIG. 1

# FIG. 2

| | HPLC CONDITIONS FOR NAC | HPLC CONDITIONS FOR NAL |
|---|---|---|
| UV<br>281 nm | | |
| FLUORESCENCE<br><br>EXCITATION 284 nm<br><br>FLUORESCENCE 333 nm | | |

EP 3 845 887 B1

# FIG. 3

| | HPLC CONDITIONS FOR NAC | HPLC CONDITIONS FOR NAL |
|---|---|---|
| UV<br><br>281 nm | | |
| FLUORESCENCE<br><br>EXCITATION 284 nm<br><br>FLUORESCENCE 333 nm | | |

EP 3 845 887 B1

# FIG. 4

| | 1/1000 | |
| --- | --- | --- |
| | HPLC CONDITIONS FOR NAC | HPLC CONDITIONS FOR NAL |
| UV 281 nm | | |
| FLUORESCENCE EXCITATION 284 nm FLUORESCENCE 333 nm | | |

EP 3 845 887 B1

FIG. 5

| | 1/1 | |
|---|---|---|
| | NAC | NAL |
| UV<br><br>281 nm | | |
| FLUORESCENCE<br><br>EXCITATION 284 nm<br><br>FLUORESCENCE 333 nm | | |

EP 3 845 887 B1

# FIG. 6

EP 3 845 887 B1

# FIG. 7

| | 1/100 | |
|---|---|---|
| | NAC | NAL |
| UV<br><br>281 nm | | |
| FLUORESCENCE<br><br>EXCITATION 284 nm<br><br>FLUORESCENCE 333 nm | | |

EP 3 845 887 B1

FIG. 8

# FIG. 9

| | UNDILUTED SOLUTION | |
| :---: | :---: | :---: |
| | HPLC CONDITIONS FOR NAC | HPLC CONDITIONS FOR NAL |
| UV<br><br>281 nm | | |
| FLUORESCENCE<br><br>EXCITATION 284 nm<br><br>FLUORESCENCE 333 nm | | |

EP 3 845 887 B1

# FIG. 10

EP 3 845 887 B1

FIG. 11

FIG. 12

# FIG. 13

| | 1/1 | |
|---|---|---|
| | HPLC CONDITIONS FOR NAC | HPLC CONDITIONS FOR NAL |
| UV<br><br>281 nm | | |
| FLUORESCENCE<br><br>EXCITATION 284 nm<br><br>FLUORESCENCE 333 nm | | |

EP 3 845 887 B1

FIG. 14

## FIG. 15

EP 3 845 887 B1

## FIG. 16

FIG. 17

| | 1/1 | |
|---|---|---|
| | HPLC CONDITIONS FOR NAC | HPLC CONDITIONS FOR NAL |
| UV<br><br>281 nm | | |
| FLUORESCENCE<br><br>EXCITATION 284 nm<br><br>FLUORESCENCE 333 nm | | |

EP 3 845 887 B1

# FIG. 18

| HPLC CONDITIONS FOR NAC | HPLC CONDITIONS FOR NAL |
|---|---|

UV

281 nm

FLUORESCENCE

EXCITATION 284 nm

FLUORESCENCE 333 nm

EP 3 845 887 B1

FIG. 19

# FIG. 20

EP 3 845 887 B1

FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011059102 A

- JP 2014037995 A

**Non-patent literature cited in the description**

- **GERBERICK, G. F. et al.** Development of a peptide reactivity assay for screening contact allergens. *Toxicological Sciences,* 2004, vol. 81 (2), 332-43
- **GERBERICK, G. F. et al.** Quantification of chemical peptide reactivity for screening contact allergens: a classification tree model approach. *Toxicological Sciences,* 2007, vol. 97 (2), 417-27

- **D. T. LOOTS ; F. H. VAN DER WESTHUIZEN ; L. BOTES.** Aloe ferox leaf gel phytochemical content, antioxidant capacity, and possible health benefits. *Journal of Agricultural and Food Chemistry,* 2007, vol. 55, 6891-6896